# EUROPEAN PATENT APPLICATION

(11) **EP 4 475 257 A1**
(43) Date of publication of application: **11.12.2024**
(21) Application number: 23749872.0
(22) Date of filing: 03.02.2023
(51) Int. Cl.: H01M 10/0567, C07C 317/40, C07F 9/40, H01M 10/052, H01M 10/054, H01M 10/0568, H01M 10/0569, H01M 10/058

(54) **NON-AQUEOUS ELECTROLYTE, NON-AQUEOUS ELECTROLYTE BATTERY, NON-AQUEOUS ELECTROLYTE BATTERY PRODUCTION METHOD, COMPOUND, AND ADDITIVE FOR NON-AQUEOUS ELECTROLYTE**

(30) Priority: 04.02.2022 JP 2022016731
(71) Applicant: Central Glass Company, Limited, Ube-shi, Yamaguchi 755-0001 (JP)
(72) Inventor: IWASAKI, Susumu, Tokyo 101-0054 (JP); KATAOKA, Fuga, Tokyo 101-0054 (JP); MORINAKA, Takayoshi, Tokyo 101-0054 (JP)
(74) Representative: Manitz Finsterwald Patent- und Rechtsanwaltspartnerschaft mbB
(86) International application number: PCT/JP2023/003631
(87) International publication number: WO 2023/149556

(57) **Abstract**

The present disclosure provides a nonaqueous electrolyte solution containing: (1) at least one selected from the group consisting of a compound represented by Formula (1) described in the specification, and a compound represented by Formula (2) described in the specification; a nonaqueous electrolyte solution battery including at least a positive electrode, a negative electrode, and the nonaqueous electrolyte solution; and a method for producing a nonaqueous electrolyte solution battery using the nonaqueous electrolyte solution.

## Description

### TECHNICAL FIELD

The present disclosure relates to a nonaqueous electrolyte solution, a nonaqueous electrolyte solution battery, a method for producing the nonaqueous electrolyte solution battery, a compound, and an additive for a nonaqueous electrolyte solution.

### BACKGROUND ART

As measures for improving cycle characteristics, storability at a high temperature, and durability of a nonaqueous electrolyte solution battery, optimization of various constituents of the battery such as active materials of a positive electrode and a negative electrode has been hitherto studied. Also, optimization of techniques relating to a nonaqueous electrolyte solution has been studied, and a variety of additives to prevent deterioration of the electrolyte solution due to decomposition of the electrolyte solution on surfaces of active positive and negative electrodes have been proposed.

For example, Patent Literature 1 proposes that various battery characteristics such as storage characteristics at a high temperature are improved by adding vinylene carbonate to an electrolyte solution. This method is to prevent the electrolyte solution from decomposing on an electrode surface by coating an electrode with a polymer film formed by polymerization of vinylene carbonate, but this causes a problem that lithium ions have difficulty in passing through the film so that internal resistance is increased and a lot of gas is generated during storage at a high temperature.

Addition of lithium difluorophosphate disclosed in Patent Literature 2 is effective to solve this problem, and it is known that the use of vinylene carbonate and lithium difluorophosphate together can provide a battery in which an increase in internal resistance and generation of gas are suppressed and that has a good storage characteristics at a high temperature.

For a simple additive instead of a combination of two or more additives, Patent Literature 3 discloses a nonaqueous electrolyte solution having a specific carbamyl phosphonate derivative having a dialkyl carbamyl group as a nonaqueous electrolyte solution having relatively good charge and discharge efficiency and exhibiting flame retardancy.

Patent Literature 4 discloses that adding a cyclic phosphoramidate carbonyl derivative having a specific polar group to a nonaqueous electrolyte solution achieves improvement of electrochemical properties in a wide temperature range, such as low-temperature discharge properties after storage at a high temperature.

Patent Literature 5 discloses that a nonaqueous electrolyte solution and a lithium battery achieve excellent battery characteristics such as a storage characteristic of a primary battery and a high temperature cycle characteristic when a secondary battery is used at a high voltage, by containing 0.01% by mass to 10% by mass of a specific sulfone compound having a sulfonylaminocarbonyl structure in the nonaqueous electrolyte solution in which an electrolyte salt is dissolved in a nonaqueous solvent.

### CITATION LIST

### PATENT LITERATURE

Patent Literature 1: JP3438636B
Patent Literature 2: JP3439085B
Patent Literature 3: JPH10-189039A
Patent Literature 4: JP6572897B
Patent Literature 5: JP5428274B

### SUMMARY OF INVENTION

### TECHNICAL PROBLEM

However, as a result of the examination, the present inventors have found that an addition of these carbamyl phosphonate derivatives or sulfone compounds causes an increase in internal resistance and causes no or a small effect of improving initial input and output characteristics. Thus, there is room for further improvement of resistance characteristics, particularly of initial resistance characteristics.

The present disclosure is made in view of the above circumstances, and an object thereof is to provide a nonaqueous electrolyte solution and a nonaqueous electrolyte solution battery having a low initial resistance value, and a method for producing the nonaqueous electrolyte solution battery having a low initial resistance value. Further, another object is to provide a compound and an additive for a nonaqueous electrolyte solution, both of which can be suitably used in the above nonaqueous electrolyte solution.

### SOLUTION TO PROBLEM

The present inventors conducted intensive research in view of such a problem and found that a nonaqueous electrolyte solution battery having a low initial resistance value can be obtained by using a nonaqueous electrolyte solution containing: (I) at least one selected from the group consisting of a compound represented by Formula (1) to be described later and a compound represented by Formula (2) to be described later (hereinafter, also described as "component (I)"), thereby solving the above problem.

That is, the present inventors have found that the above problem can be solved by the following configurations.
[1] A nonaqueous electrolyte solution containing:
   (I) at least one selected from the group consisting of a compound represented by the following Formula (1) and a compound represented by the following Formula (2). 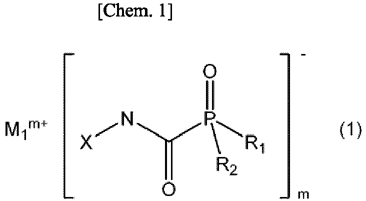
      (In Formula (1), R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R₁ and R₂ optionally bond to each other to form a cyclic structure;
      X represents a monovalent substituent; and
      M₁^{m+} represents a proton, a metal cation, or an onium cation, and m represents a valence of the cation. 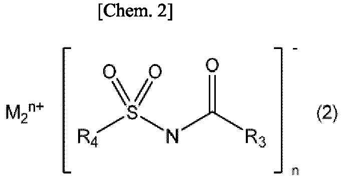
      (In Formula (2), R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
      R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
      M₂ⁿ⁺ represents a proton, a metal cation, or an onium cation, and n represents a valence of the cation).
[2] The nonaqueous electrolyte solution according to [1], in which
   in the Formula (1), X is -C(=O)-Rₐ, -S(=O)₂-R_{b}, or -P(=O)-R_{c}R_{d}, Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms (at least one hydrogen atom in the organic group is optionally substituted with a halogen atom, and R_{c} and R_{d} optionally bond to each other to form a cyclic structure).
[3] The nonaqueous electrolyte solution according to [1] or [2], in which
   in the Formula (1), X represents -S(=O)₂F or -P(=O)F₂.
[4] The nonaqueous electrolyte solution according to any one of [1] to [3], in which
   at least one selected from the group consisting of M₁^{m+} in the Formula (1) and M₂ⁿ⁺ in the Formula (2) represents a lithium ion, a sodium ion, or a proton.
[5] The nonaqueous electrolyte solution according to any one of [1] to [4], in which
   at least one selected from the group consisting of the compound represented by the Formula (1) and the compound represented by the Formula (2) contains at least one carbon-carbon unsaturated bond.
[6] The nonaqueous electrolyte solution according to any one of [1] to [5], in which
   the nonaqueous electrolyte solution contains a (II) solute, and
   the (II) solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and Nal.
[7] The nonaqueous electrolyte solution according to any one of [1] to [6], in which
   the nonaqueous electrolyte solution contains a (III) nonaqueous organic solvent, and
   the (III) nonaqueous organic solvent contains at least one selected from the group consisting of a cyclic carbonate and a chain carbonate.
[8] The nonaqueous electrolyte solution according to [7], in which
   the cyclic carbonate is at least one selected from the group consisting of ethylene carbonate, propylene carbonate, and fluoroethylene carbonate, and the chain carbonate is at least one selected from the group consisting of ethyl methyl carbonate, dimethyl carbonate, diethyl carbonate, and methyl propyl carbonate.
[9] The nonaqueous electrolyte solution according to any one of [1] to [8], in which
   a content of the (I) is 0.01% by mass to 10.0% by mass with respect to a total amount of the nonaqueous electrolyte solution.
[10] The nonaqueous electrolyte solution according to any one of [1] to [9], further containing:
   at least one selected from vinylene carbonate, bis(oxalato)borate, difluorooxalatoborate, difluorobis(oxalato)phosphate, tetrafluorooxalatophosphate, bis(fluorosulfonyl)imide salt, bis(difluorophosphoryl)imide salt, (difluorophosphoryl)(fluorosulfonyl)imide salt, difluorophosphate, fluorosulfonate, methanesulfonyl fluoride, 1,3,2-dioxathiolane-2,2-dioxide, 1,3-propenesultone, 1,3-propanesultone, 1,6-diisocyanatohexane, ethynylethylene carbonate, 4-propyl-1,3,2-dioxathiolane-2,2-dioxide, methylene methanedisulfonate, 1,2-ethanedisulfonic anhydride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.
[11] A nonaqueous electrolyte solution battery including:
   at least a positive electrode, a negative electrode, and the nonaqueous electrolyte solution according to any one of [1] to [10].
[12] A method for producing a nonaqueous electrolyte solution battery, the method including:
   preparing the nonaqueous electrolyte solution according to any one of [1] to [10]; and
   filling an empty cell including a positive electrode and a negative electrode with the nonaqueous electrolyte solution.
[13] A compound represented by the following Formula (1A). 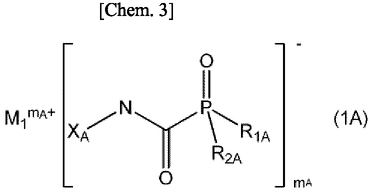
   (In Formula (1A), R_{1A} and R_{2A} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R_{1A} and R_{2A} optionally bond to each other to form a cyclic structure;
   X_{A} represents a monovalent substituent; and
   M₁^{mA+} represents a metal cation or an onium cation, and m_{A} represents a valence of the cation).
[14] The compound according to [13], in which
   in the Formula (1A), M₁^{mA+} represents a lithium ion or a sodium ion.
[15] The compound according to [13] or [14], in which
   the compound represented by the Formula (1A) contains at least one carbon-carbon unsaturated bond.
[16] A compound represented by the following Formula (1B). 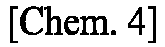 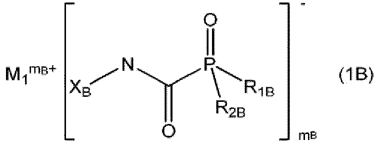
   (In Formula (1B), R_{1B} and R_{2B} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R_{1B} and R_{2B} optionally bond to each other to form a cyclic structure;
   X_{B} represents a monovalent substituent;
   M₁^{mB+} represents a proton, a metal cation, or an onium cation, and ms represents a valence of the cation; and
   the compound represented by Formula (1B) contains at least one carbon-carbon unsaturated bond).
[17] The compound according to any one of [13] to [16], in which
   at least one selected from the group consisting of X_{A} in the Formula (1A) and X_{B} in the Formula (1B) is -C(=O)-Rₐ, -S(=O)₂-R_{b}, or -P(=O)-R_{c}R_{d}, and Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms (at least one hydrogen atom in the organic group is optionally substituted with a halogen atom, and R_{c} and R_{d} optionally bond to each other to form a cyclic structure).
[18] The compound according to any one of [13] to [17], in which
   at least one selected from the group consisting of X_{A} in the Formula (1A) and X_{B} in the Formula (1B) represents - S(=O)₂F or -P(=O)F₂.
[19] A compound represented by the following Formula (2C). 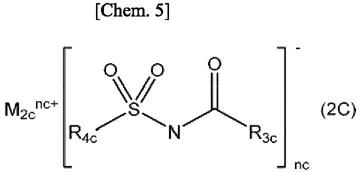
   (In Formula (2C), R_{3C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom, and
   R_{4C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom, and
   M_{2C}^{nC+} represents a proton, a metal cation, or an onium cation, and nc represents a valence of the cation).
[20] A compound represented by the following Formula (2D). 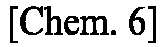 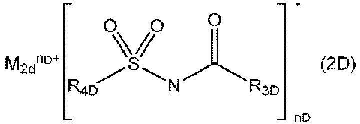
   (In Formula (2D), R_{3D} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom, and
   R_{4D} represents a fluorine atom; and
   M_{2d}^{nD+} represents a proton, a metal cation, or an onium cation, and n_{D} represents a valence of the cation).
[21] The compound according to [19], in which
   M_{2C}^{nC+} in the Formula (2C) represents a lithium ion, a sodium ion, or a proton.
[22] The compound according to [20], in which
   M_{2d}^{nD+} in the Formula (2D) represents a lithium ion or a sodium ion.
[23] An additive for a nonaqueous electrolyte solution, including:
   at least one selected from the group consisting of a compound represented by the following Formula (1) and a compound represented by the following Formula (2). 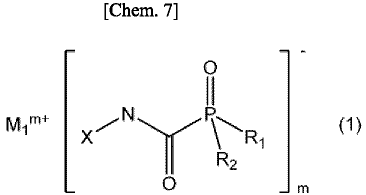
   (In Formula (1), R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R₁ and R₂ optionally bond to each other to form a cyclic structure;
   X represents a monovalent substituent; and
   M₁^{m+} represents a proton, a metal cation, or an onium cation, and m represents a valence of the cation). 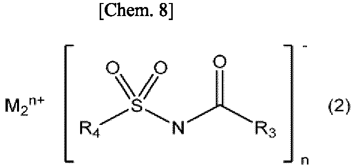
   (In Formula (2), R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
   R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
   M₂ⁿ⁺ represents a proton, a metal cation, or an onium cation, and n represents a valence of the cation).

### ADVANTAGEOUS EFFECTS OF INVENTION

The present disclosure can provide a nonaqueous electrolyte solution and a nonaqueous electrolyte solution battery having a low initial resistance value, and a method for producing a nonaqueous electrolyte solution battery having a low initial resistance value. In addition, the present disclosure can provide a compound and an additive for a nonaqueous electrolyte solution, both of which can be suitably used in the above nonaqueous electrolyte solution.

### DESCRIPTION OF EMBODIMENTS

Configurations and combinations thereof in the following embodiments are merely examples, and additions, replacements, and other changes of the configurations may be made without departing from the scope of the present disclosure. In addition, the present disclosure is not limited by the embodiments, but only by the scope of claims.

In the present description, the expression "to" means to include numerical values before and after "to" as a lower limit value and an upper limit value, respectively.

An initial resistance value in the present specification means a resistance value of a nonaqueous electrolyte solution battery immediately after an initial charge and discharge operation for battery stabilization. Specifically, the initial resistance value refers to a resistance value obtained by initial impedance measurement after three cycles of a charge and discharge operation for battery stabilization.

### [1. Nonaqueous Electrolyte Solution]

A nonaqueous electrolyte solution according to the present disclosure is a nonaqueous electrolyte solution containing (I) at least one selected from the group consisting of a compound represented by the above Formula (1) and a compound represented by the above Formula (2).

### <Component (I)>

The nonaqueous electrolyte solution according to the present disclosure contains at least one selected from the group consisting of a compound represented by Formula (1) and a compound represented by Formula (2), which represent as the component (I).

When the nonaqueous electrolyte solution containing the above component (I) is used in a nonaqueous electrolyte solution battery (for example, a lithium ion secondary battery or a sodium ion secondary battery), the component (I) decomposes on at least one of a positive electrode and a negative electrode, and forms a film having good cation conductivity on a surface of at least one of the positive electrode and the negative electrode. It is considered that this film prevents direct contact between a nonaqueous organic solvent or solute and an electrode active material and reduces cation dissociation energy of the solute. The present inventors assume that an effect of reducing initial resistance of the nonaqueous electrolyte solution battery is exhibited as a result of the above.

The compound represented by Formula (1) is described below.

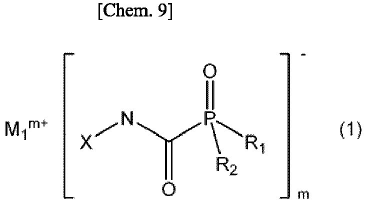

In Formula (1), R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group is optionally substituted with a halogen atom. R₁ and R₂ optionally bond to each other to form a cyclic structure. X represents a monovalent substituent. M₁^{m+} represents a proton, a metal cation, or an onium cation. m represents a valence of the cation.

In Formula (1), R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms.

As for the alkoxy group having 1 to 10 carbon atoms in R₁ and R₂, examples of an alkyl group in the alkoxy group include a methyl group, an ethyl group, an n-propyl group, an isopropyl group, an n-butyl group, a sec-butyl group, an isobutyl group, a tert-butyl group, an n-pentyl group, an n-hexyl group, an n-octyl group, and an n-decanyl group.

As for the alkenyloxy group having 2 to 10 carbon atoms in R₁ and R₂, examples of an alkenyl group in the alkenyloxy group include a vinyl group, an allyl group, a 1-propenyl group, an isopropenyl group, a 2-butenyl group, and a 1,3-butadienyl group.

As for the alkynyloxy group having 2 to 10 carbon atoms in R₁ and R₂, examples of an alkynyl group of the alkynyloxy group include an ethynyl group, a 2-propynyl group, and a 1,1-dimethyl-2-propynyl group.

As for the cycloalkoxy group having 3 to 10 carbon atoms in R₁ and R₂, examples of a cycloalkyl group of the cycloalkoxy group include a cyclopentyl group and a cyclohexyl group.

As for the cycloalkenyloxy group having 3 to 10 carbon atoms in R₁ and R₂, examples of a cycloalkenyl group of the cycloalkenyloxy group include a cyclopentenyl group and a cyclohexenyl group.

As for the aryloxy group having 6 to 10 carbon atoms in R₁ and R₂, examples of an aryl group of the aryloxy group include a phenyl group, a tolyl group, and an xylyl group.

At least one of any hydrogen atoms of the above organic group is optionally substituted with a halogen atom. Examples of the halogen atom include a fluorine atom, a chlorine atom, a bromine atom, and an iodine atom, and a fluorine atom is preferable.

The above R₁ and R₂ optionally bond to each other to form a cyclic structure. The cyclic structure formed by R₁ and R₂ together with a phosphorus atom is preferably a 5- or 6-membered ring, more preferably a 5-membered ring.

R₁ and R₂ are each preferably independently an alkoxy group having 1 to 3 carbon atoms, an alkenyloxy group having 2 to 4 carbon atoms, or an alkynyloxy group having 2 to 4 carbon atoms, more preferably a methoxy group, a vinyloxy group, an allyloxy group, or a 2-propynyloxy group, and still more preferably a vinyloxy group, an allyloxy, or a 2-propynyloxy group.

Preferably, R₁ and R₂ represent the alkoxy group having 1 or 2 carbon atoms and bond to each other to form a 5- or 6-membered ring with a phosphorus atom.

As described later, the compound represented by Formula (1) preferably contains at least one carbon-carbon unsaturated bond, and at least one of R₁ and R₂ is preferably a group containing a carbon-carbon unsaturated bond.

In Formula (1), X represents a monovalent substituent.

The monovalent substituent represented by X is not limited but is preferably -C(=O)-Rₐ, -S(=O)₂-R_{b}, or -P(=O)-R_{c}R_{d}. Preferably, Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms (at least one hydrogen atom in the organic group is optionally substituted with a halogen atom. R_{c} and R_{d} optionally bond to each other to form a cyclic structure).

As for the alkyl group having 1 to 8 carbon atoms in Rₐ to R_{d}, examples of the alkyl group include a methyl group, an ethyl group, an n-propyl group, an isopropyl group, an n-butyl group, a sec-butyl group, an isobutyl group, a tert-butyl group, an n-pentyl group, an n-hexyl group, and an n-octyl group.

As for the alkoxy group having 1 to 8 carbon atoms in Rₐ to R_{d}, examples of an alkyl group in the alkoxy group include alkyl groups having 1 to 8 carbon atoms represented by Rₐ to R_{d}.

As for the alkenyl group having 2 to 6 carbon atoms in Rₐ to R_{d}, examples of the alkenyl group include a vinyl group, an allyl group, a 1-propenyl group, an isopropenyl group, a 2-butenyl group, and a 1,3-butadienyl group.

As for the alkenyloxy group having 2 to 6 carbon atoms in Rₐ to R_{d}, examples of an alkenyl group in the alkenyloxy group include an alkenyl group having 2 to 6 carbon atoms represented by Rₐ to R_{d}.

As for the alkynyl group having 3 to 6 carbon atoms in Rₐ to R_{d}, examples of the alkynyl group include a 2-propynyl group and a 1,1-dimethyl-2-propynyl group.

As for the alkynyloxy group having 3 to 6 carbon atoms in Rₐ to R_{d}, examples of an alkynyl group in the alkynyloxy group include an alkynyl group having 3 to 6 carbon atoms represented by Rₐ to R_{d}.

As for the aryl group having 6 to 12 carbon atoms in Rₐ to R_{d}, examples of the aryl group include a phenyl group, a tolyl group, and an xylyl group.

As for the aryloxy group having 6 to 12 carbon atoms in Rₐ to R_{d}, examples of an aryl group in the aryloxy group include an aryl group having 6 to 12 carbon atoms represented by Rₐ to R_{d}.

At least one of any hydrogen atoms of the above organic group is optionally substituted with a halogen atom. Examples of the halogen atom include a fluorine atom, a chlorine atom, a bromine atom, and an iodine atom, and a fluorine atom is preferable.

The above R_{c} and R_{d} optionally bond to each other to form a cyclic structure. The cyclic structure formed by R_{c} and R_{d} together with a phosphorus atom is preferably a 5- or 6-membered ring, and more preferably a 5-membered ring.

When X represents -C(=O)-Rₐ, Rₐ preferably represents an alkyl group having 1 to 3 carbon atoms, an alkoxy group having 1 to 3 carbon atoms, an alkenyloxy group having 2 to 4 carbon atoms, an alkynyloxy group having 3 to 5 carbon atoms, an aryl group having 6 to 8 carbon atoms, or an aryloxy group having 6 to 8 carbon atoms, more preferably a methyl group, a methoxy group, an allyloxy group, or a 2-propynyloxy group, and still more preferably an allyloxy group or a 2-propynyloxy group.

When X represents -S(=O)₂-R_{b}, R_{b} preferably represents a fluorine atom, an alkyl group having 1 to 3 carbon atoms, an alkoxy group having 1 to 3 carbon atoms, an alkenyloxy group having 2 to 3 carbon atoms, an alkynyloxy group having 3 to 5 carbon atoms, or an aryl group having 6 to 8 carbon atoms, more preferably a fluorine atom, a methyl group, a methoxy group, an allyloxy group, or a 2-propynyloxy group, and still more preferably a fluorine atom.

When X represents -P(=O)-R_{c}R_{d}, R_{c} and R_{d} each independently represent a fluorine atom, an alkoxy group having 1 to 3 carbon atoms, an alkenyloxy group having 2 to 3 carbon atoms, or an alkynyloxy group having 3 to 5 carbon atoms, more preferably a fluorine atom or an alkoxy group having 1 to 3 carbon atoms, and still more preferably a fluorine atom or a methoxy group.

Preferably, R_{c} and R_{d} represent an alkoxy group having 1 or 2 carbon atoms and bond to each other with a phosphorus atom to form a 5-membered ring or 6-membered ring.

In the Formula (1), particularly preferably, X represents -S(=O)₂F or -P(=O)F₂.

As described later, the compound represented by Formula (1) preferably contains at least one carbon-carbon unsaturated bond, and X is preferably a group containing a carbon-carbon unsaturated bond.

In Formula (1), M₁^{m+} represents a proton, a metal cation, or an onium cation. m represents a valence of the cation.

When M₁^{m+} represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M₁^{m+} represents an onium cation, examples of the onium cation include a tetraalkylammonium ion.
m represents the valence of the cation. When M₁^{m+} represents a cation having a valence of 1, m is 1, and when M₁^{m+} represents a cation having a valence of 2, m is 2.

M₁^{m+} preferably represents a proton or a metal cation, and more preferably represents a lithium ion, a sodium ion, or a proton. When the nonaqueous electrolyte solution is used in a lithium ion battery, a proton or a lithium ion is more preferable, and when the nonaqueous electrolyte solution is used in a sodium ion battery, a proton or a sodium ion is more preferable.

The compound represented by the Formula (1) preferably contains at least one carbon-carbon unsaturated bond, and more preferably 1 to 2 carbon-carbon unsaturated bonds, from the viewpoint of further improving a discharge capacity maintenance rate with the initial resistance value kept low.

Specific examples of the compound represented by Formula (1) are shown below, but the present invention is not limited thereto. In the structural formulae to be described later, Me represents a methyl group and Et represents an ethyl group.

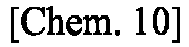

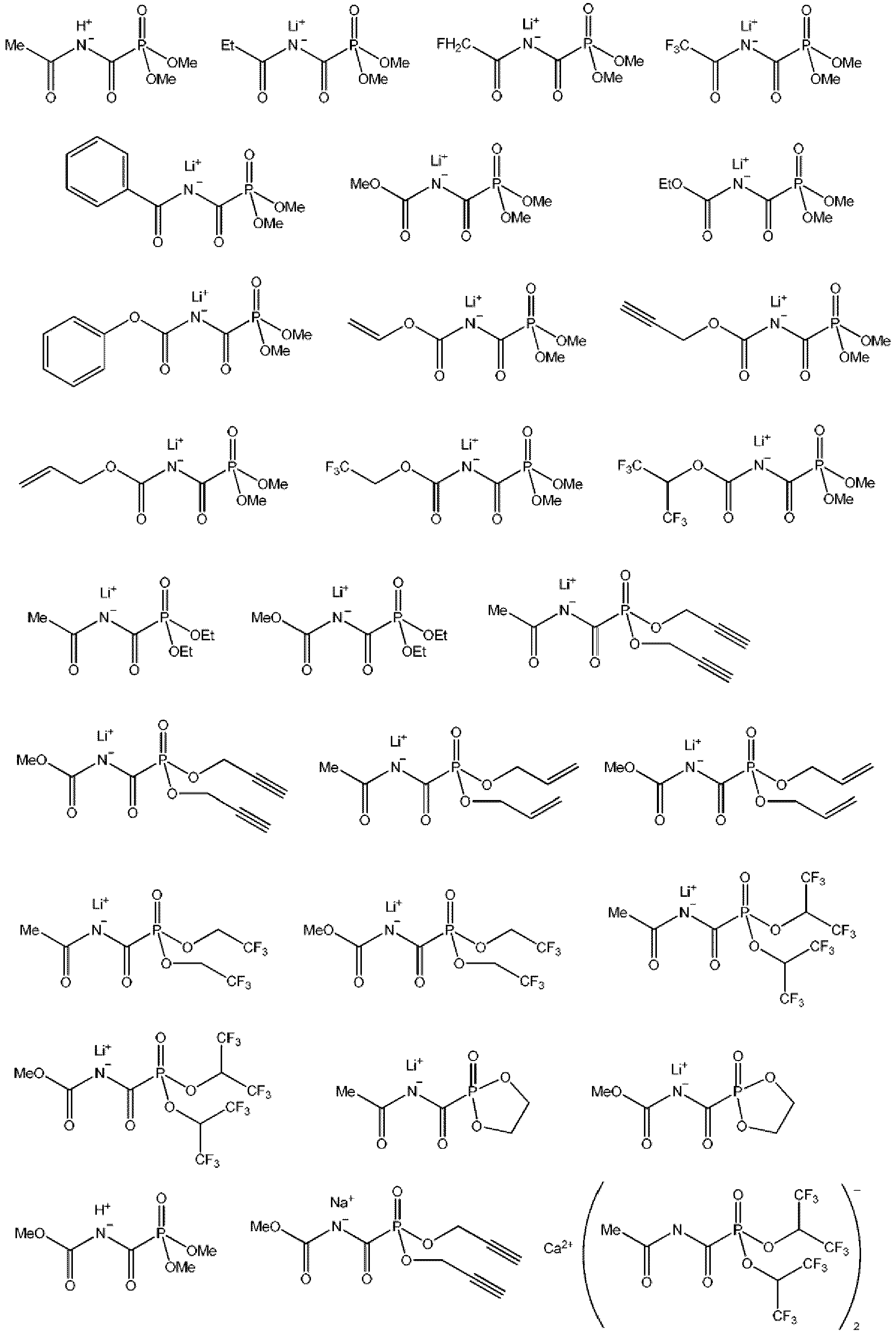

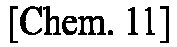

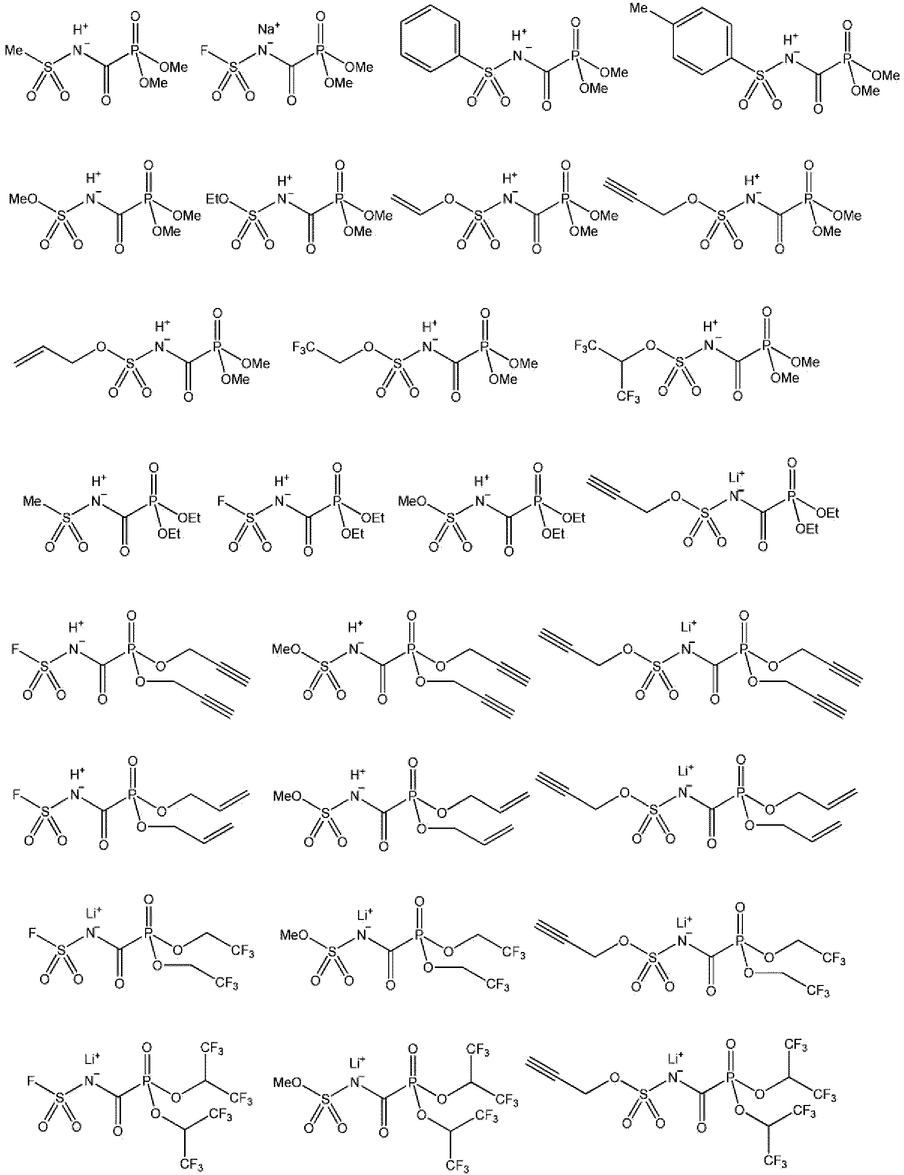

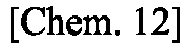

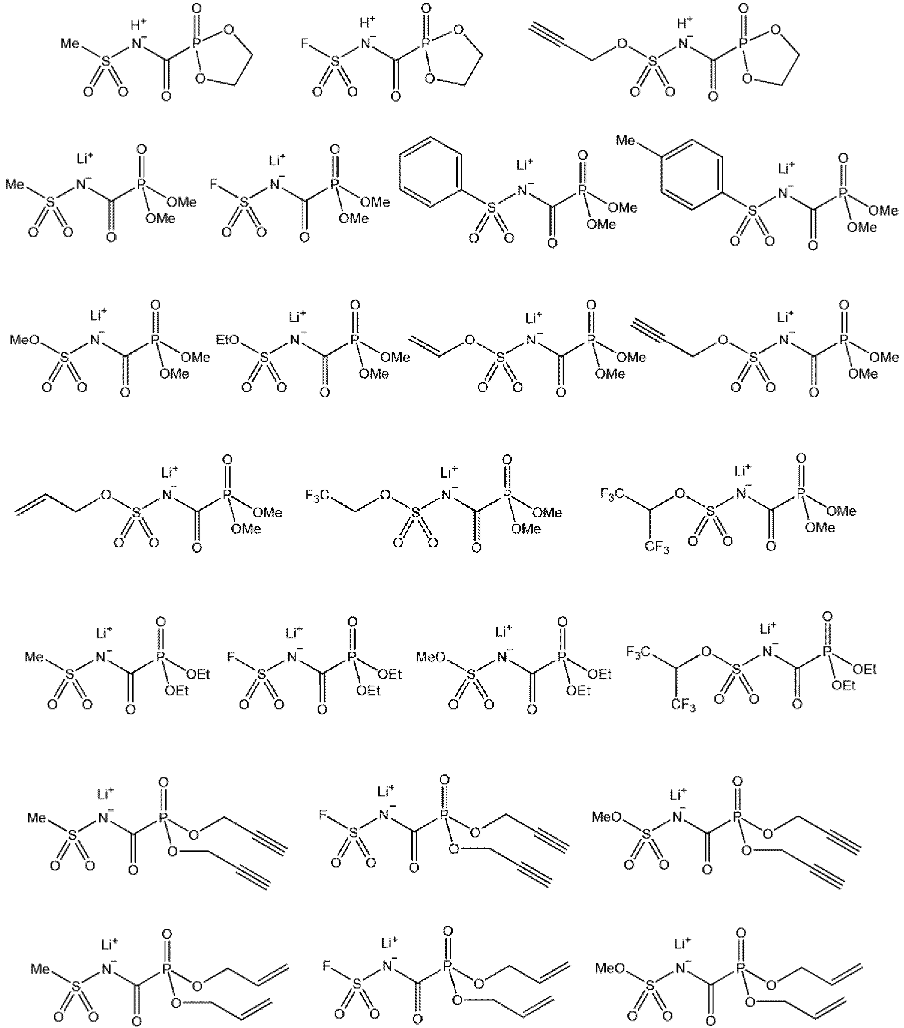

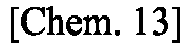

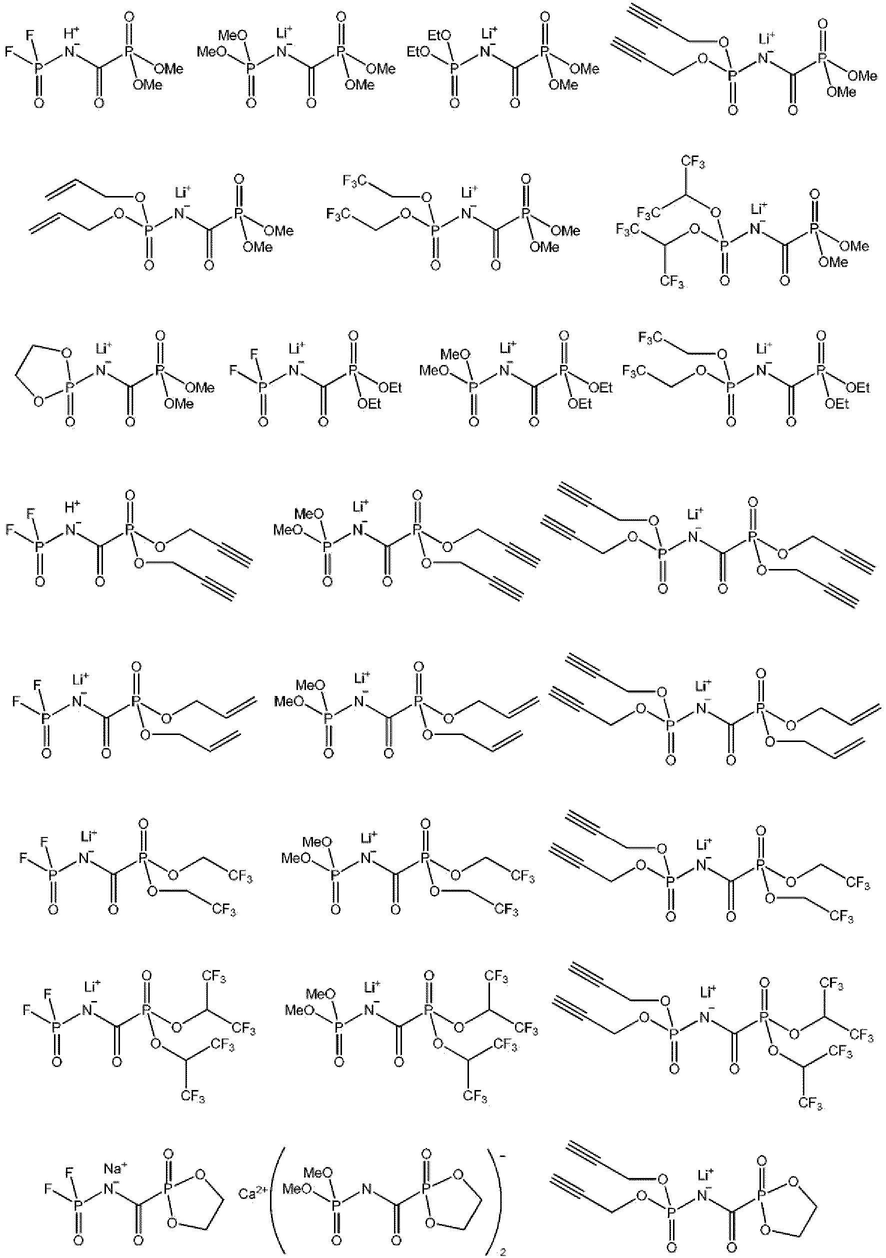

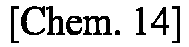

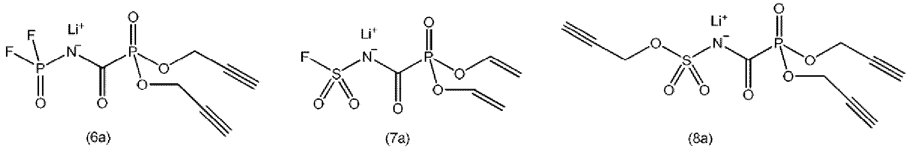

Next, the compound represented by Formula (2) is described.

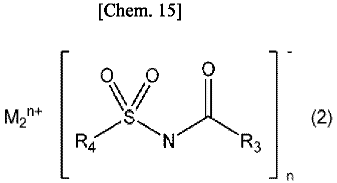

In Formula (2), R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group is optionally substituted with a halogen atom. R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group is optionally substituted with a halogen atom. M₂ⁿ⁺ represents a proton, a metal cation, or an onium cation, n represents the valence of the cation.

In Formula (2), R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms.

Specific examples in R₃ of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, include those examples described above in R₁ and R₂ for the alkoxy group having 1 to 10 carbon atoms, the alkenyloxy group having 2 to 10 carbon atoms, the alkynyloxy group having 2 to 10 carbon atoms, the cycloalkoxy group having 3 to 10 carbon atoms, the cycloalkenyloxy group having 3 to 10 carbon atoms, and the aryloxy group having 6 to 10 carbon atoms.

At least one of any hydrogen atoms of the above organic group is optionally substituted with a halogen atom. Examples of the halogen atom include a fluorine atom, a chlorine atom, a bromine atom, and an iodine atom, a fluorine atom or a chlorine atom is preferable, and a fluorine atom is more preferable.

R₃ is preferably an alkoxy group having 1 to 3 carbon atoms, an alkenyloxy group having 2 to 4 carbon atoms, or an alkynyloxy group having 2 to 4 carbon atoms, more preferably a methoxy group, a vinyloxy group, an allyloxy group, or a 2-propynyloxy group, and still more preferably a vinyloxy group, an allyloxy group, or a 2-propynyloxy group.

As described later, the compound represented by Formula (2) preferably contains at least one carbon-carbon unsaturated bond, and R₃ is preferably a group containing a carbon-carbon unsaturated bond.

In Formula (2), R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms.

When R₄ represents a halogen atom, examples of the halogen atom include a fluorine atom, a chlorine atom, a bromine atom, and an iodine atom, a fluorine atom or a chlorine atom is preferable, and a fluorine atom is more preferable.

Specific examples of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, which are represented by R₄, include those examples described above in R₁ and R₂ for the alkoxy group having 1 to 10 carbon atoms, the alkenyloxy group having 2 to 10 carbon atoms, the alkynyloxy group having 2 to 10 carbon atoms, the cycloalkoxy group having 3 to 10 carbon atoms, the cycloalkenyloxy group having 3 to 10 carbon atoms, and the aryloxy group having 6 to 10 carbon atoms.

At least one of any hydrogen atoms of the above organic group is optionally substituted with a halogen atom. Examples of the halogen atom include a fluorine atom, a chlorine atom, a bromine atom, and an iodine atom, a fluorine atom or a chlorine atom is preferable, and a fluorine atom is more preferable.

R₄ is preferably a fluorine atom, a chlorine atom, an alkoxy group having 1 to 3 carbon atoms, an alkenyloxy group having 2 to 4 carbon atoms, or an alkynyloxy group having 2 to 4 carbon atoms, more preferably a fluorine atom, a methoxy group, a vinyloxy group, an allyloxy group, or a 2-propynyloxy group, and still more preferably a fluorine atom, a vinyloxy group, an allyloxy group, or a 2-propynyloxy group.

As described later, the compound represented by Formula (2) preferably contains at least one carbon-carbon unsaturated bond, and R₄ is preferably a group containing a carbon-carbon unsaturated bond.

In Formula (2), M₂ⁿ⁺ represents a proton, a metal cation, or an onium cation, n represents the valence of the cation.

When M₂ⁿ⁺ represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M₂ⁿ⁺ represents an onium cation, examples of the onium cation include a tetraalkylammonium ion.
n represents the valence of the cation. When M₂ⁿ⁺ represents a cation having a valence of 1, n is 1, and when M₂ⁿ⁺ represents a cation having a valence of 2, n is 2.

M₂ⁿ⁺ preferably represents a proton or a metal cation, and more preferably represents a lithium ion, a sodium ion, or a proton. When the nonaqueous electrolyte solution is used in a lithium ion battery, a proton or a lithium ion is more preferable, and when the nonaqueous electrolyte solution is used in a sodium ion battery, a proton or a sodium ion is more preferable.

The compound represented by the Formula (2) preferably contains at least one carbon-carbon unsaturated bond, and more preferably 1 to 2 carbon-carbon unsaturated bonds, from the viewpoint of further improving a discharge capacity maintenance rate with the initial resistance value kept low.

Specific examples of the compound represented by Formula (2) are shown below, but the present invention is not limited thereto. In the structural formulae to be described later, Me represents a methyl group, Et represents an ethyl group, and Pr represents an n-propyl group.

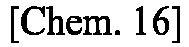

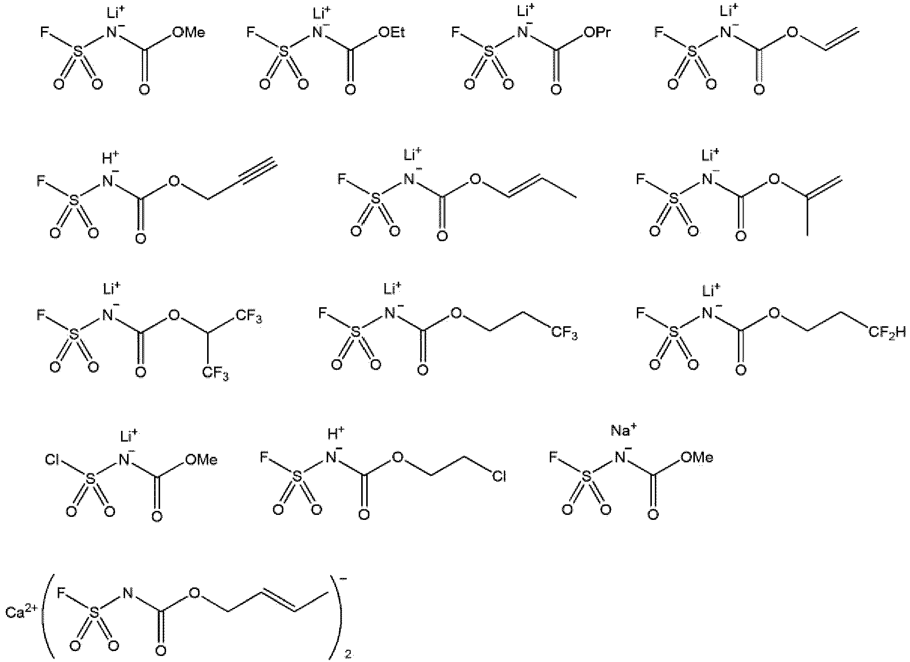

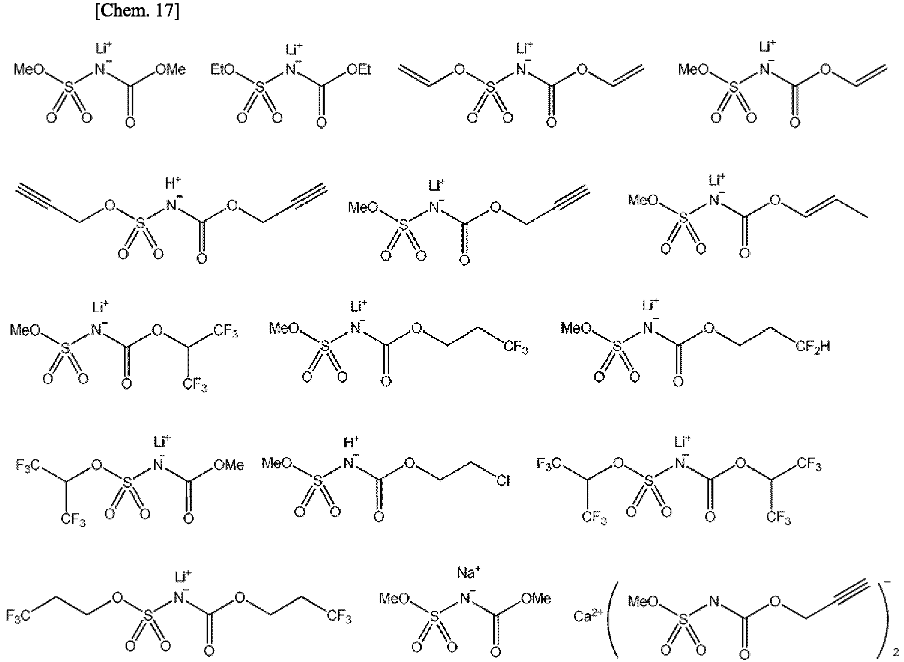

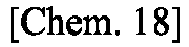

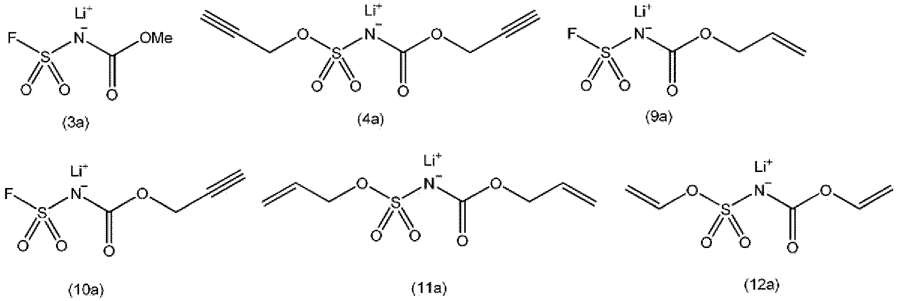

In a preferred embodiment, at least one selected from the group consisting of M₁^{m+} in the Formula (1) and M₂ⁿ⁺ in the Formula (2) preferably represents a lithium ion, a sodium ion, or a proton.

In a preferred embodiment, at least one selected from the group consisting of the compound represented by the Formula (1) and the compound represented by the Formula (2) preferably contains at least one carbon-carbon unsaturated bond.

In the nonaqueous electrolyte solution according to the present disclosure, a content of the above component (I) (hereafter, also described as "concentration of (I)") with respect to a total amount (100% by mass) of the nonaqueous electrolyte solution is preferably 0.01% by mass or more, more preferably 0.05% by mass or more, and further preferably 0.1% by mass or more. The concentration of (I) is preferably 10.0% by mass or less, more preferably 8.0% by mass or less, further preferably 4.0% by mass or less, and particularly preferably 2.5% by mass or less.

Setting the concentration of (I) to 0.01% by mass or more easily achieves an effect of improving cycle characteristics of a nonaqueous electrolyte solution battery using the nonaqueous electrolyte solution. On the other hand, setting the concentration of (I) to 10.0% by mass or less allows for preventing an increase in viscosity of the nonaqueous electrolyte solution, and easily achieves the effect of preventing the increase in resistance of the nonaqueous electrolyte solution battery using the nonaqueous electrolyte solution.

The content of the above component (I) with respect to the total amount of the nonaqueous electrolyte solution is preferably 0.01% by mass to 10.0% by mass.

In the nonaqueous electrolyte solution according to the present disclosure, one type of compounds in the component (I) may be used alone, or two or more types of the compounds may be mixed and used in any combination and any proportion according to an application.

The compound represented by Formula (1) can be produced by various methods. The production method is not limited.

For example, the compound can be obtained by allowing chlorosulfonyl isocyanate to react with di(2-propynyl) phosphite or diallyl phosphite, and then sequentially adding sodium fluoride and lithium hydride.

The compound represented by Formula (2) can be produced by various methods. The production method is not limited.

For example, the compound can be obtained by allowing chlorosulfonyl isocyanate to react with 2-propynyl alcohol or allyl alcohol in the presence of lithium hydride.

The present disclosure also relates to a compound represented by the following Formula (1A).

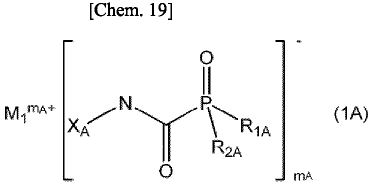

In Formula (1A), R_{1A} and R_{2A} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group is optionally substituted with a halogen atom. R_{1A} and R_{2A} optionally bond to each other to form a cyclic structure. X_{A} represents a monovalent substituent. M₁^{mA+} represents a metal cation or an onium cation. m_{A} represents the valence of the cation.

R_{1A}, R_{2A}, and X_{A} in Formula (1A) have the same meanings as R₁, R₂, and X in the Formula (1), and preferable examples thereof are also the same.

In Formula (1A), M₁^{mA+} represents a metal cation or an onium cation. m_{A} represents the valence of the cation.

When M₁^{mA+} represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M₁^{mA+} represents an onium cation, examples of the onium cation include a tetraalkylammonium ion.
m_{A} represents the valence of the cation. When M₁^{mA+} represents a cation having a valence of 1, m_{A} is 1, and when M₁^{mA+} represents a cation having a valence of 2, m_{A} is 2.

M₁^{mA+} preferably represents a metal cation, and more preferably represents a lithium ion or a sodium ion. When the nonaqueous electrolyte solution is used in a lithium ion battery, a lithium ion is more preferable, and when the nonaqueous electrolyte solution is used in a sodium ion battery, a sodium ion is more preferable.

The compound represented by the Formula (1A) preferably contains at least one carbon-carbon unsaturated bond, from the viewpoint of further improving a discharge capacity maintenance rate with the initial resistance value kept low.

The compound represented by the Formula (1A) more preferably contains 1 to 2 carbon-carbon unsaturated bonds.

The present disclosure also relates to a compound represented by the following Formula (1B).

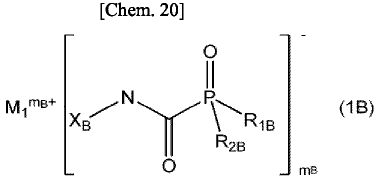

In Formula (1B), R_{1B} and R_{2B} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group is optionally substituted with a halogen atom. R_{1B} and R_{2B} optionally bond to each other to form a cyclic structure. X_{B} represents a monovalent substituent. The monovalent substituent is shown. M₁^{mB+} represents a proton, a metal cation, or an onium cation. ms represents a valence of the cation. However, the compound represented by Formula (1B) contains at least one carbon-carbon unsaturated bond.

R_{1B}, R_{2B}, and X_{B} in Formula (1B) have the same meanings as R₁, R₂, and X in the Formula (1), and preferable examples thereof are also the same.

In Formula (1B), M₁^{mB+} represents a proton, a metal cation, or an onium cation. m_{B} represents a valence of the cation.

When M₁^{mB+} represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M₁^{mB+} represents an onium cation, examples of the onium cation include a tetraalkylammonium ion.
m_{B} represents the valence of the cation. When M₁^{mB+} represents a cation having a valence of 1, m_{B} is 1, and when M₁^{mB+} represents a cation having a valence of 2, m_{B} is 2.

M₁^{mB+} preferably represents a proton or a metal cation, and more preferably represents a proton, a lithium ion, or a sodium ion. When the nonaqueous electrolyte solution is used in a lithium ion battery, a proton or a lithium ion is more preferable, and when the nonaqueous electrolyte solution is used in a sodium ion battery, a proton or a sodium ion is more preferable.

The compound represented by the Formula (1B) contains at least one carbon-carbon unsaturated bond. The compound represented by Formula (1B) more preferably contains 1 to 2 carbon-carbon unsaturated bonds.

At least one selected from the group consisting of X_{A} in the Formula (1A) and X_{B} in the Formula (1B) is -C(=O)-Rₐ, -S(=O)₂-R_{b}, or -P(=O)-R_{c}R_{d}, and preferably, Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms (at least one hydrogen atom in the organic group is optionally substituted with a halogen atom. R_{c} and R_{d} optionally bond to each other to form a cyclic structure).

At least one selected from the group consisting of X_{A} in the Formula (1A) and X_{B} in the Formula (1B) preferably represents -S(=O)₂F or -P(=O)F₂.

Specific examples of the compound represented by the Formula (1A) include the specific examples of the compound represented by the Formula (1) that are included in the compound represented by Formula (1A) (specifically, compounds in which the cation is a metal cation or an onium cation among the compounds represented by Formula (1)).

Further, specific examples of the compound represented by the Formula (1B) include the specific examples of the compound represented by the Formula (1), that are included in the compound represented by Formula (1B) (specifically, compounds containing at least one carbon-carbon unsaturated bond among the compounds represented by Formula (1)).

The present disclosure also relates to a compound represented by the following Formula (2C).

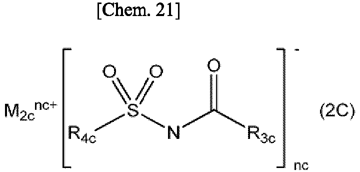

In Formula (2C), R_{3C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms. At least one hydrogen atom in the organic group is optionally substituted with a halogen atom.

R_{4C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms. At least one hydrogen atom in the organic group is optionally substituted with a halogen atom.

M_{2C}^{nC+} represents a proton, a metal cation, or an onium cation, nc represents the valence of the cation.

In Formula (2C), R_{3C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms.

Specific examples in Rsc of an alkenyloxy group having 2 to 10 carbon atoms, an alkynyloxy group having 2 to 10 carbon atoms, and a cycloalkenyloxy group having 3 to 10 carbon atoms include those examples described above in R₃ for the alkenyloxy group having 2 to 10 carbon atoms, the alkynyloxy group having 2 to 10 carbon atoms, and the cycloalkenyloxy group having 3 to 10 carbon atoms.

At least one of any hydrogen atoms of the above organic group is optionally substituted with a halogen atom. Examples of the halogen atom include a fluorine atom, a chlorine atom, a bromine atom, and an iodine atom, a fluorine atom or a chlorine atom is preferable, and a fluorine atom is more preferable.

R_{3C} is preferably an alkenyloxy group having 2 to 4 carbon atoms or an alkynyloxy group having 2 to 4 carbon atoms, and more preferably a vinyloxy group, an allyloxy group, or a 2-propynyloxy group.

In Formula (2C), R_{4C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms. Specific examples in R_{4C} of an alkenyloxy group having 2 to 10 carbon atoms, an alkynyloxy group having 2 to 10 carbon atoms, and a cycloalkenyloxy group having 3 to 10 carbon atoms include those examples described above in R₄ for the alkenyloxy group having 2 to 10 carbon atoms, the alkynyloxy group having 2 to 10 carbon atoms, and the cycloalkenyloxy group having 3 to 10 carbon atoms.

At least one of any hydrogen atoms of the above organic group is optionally substituted with a halogen atom. Examples of the halogen atom include a fluorine atom, a chlorine atom, a bromine atom, and an iodine atom, a fluorine atom or a chlorine atom is preferable, and a fluorine atom is more preferable.

R_{4C} is preferably a vinyloxy group, an allyloxy group, or a 2-propynyloxy group, and more preferably a vinyloxy group, an allyloxy group, or a 2-propynyloxy group.

In Formula (2C), M_{2C}^{nC+} represents a proton, a metal cation, or an onium cation. nc represents the valence of the cation.

When M_{2C}^{nC+} represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M_{2C}^{nC+} represents an onium cation, examples of the onium cation include a tetraalkylammonium ion.
nc represents the valence of the cation. When M_{2C}^{nC+} represents a cation having a valence of 1, nc is 1, and when M_{2C}^{nC+} represents a cation having a valence of 2, nc is 2.

M_{2C}^{nC+} preferably represents a proton or a metal cation, and more preferably represents a lithium ion, a sodium ion, or a proton. When the nonaqueous electrolyte solution is used in a lithium ion battery, a proton or a lithium ion is more preferable, and when the nonaqueous electrolyte solution is used in a sodium ion battery, a proton or a sodium ion is more preferable.

Specific examples of the compound represented by the Formula (2C) include the specific examples of the compound represented by the Formula (2) that are included in the compound represented by Formula (2C) (specifically, compounds in which R₃ is an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and R₄ is an organic group selected from the group consisting of an alkenyloxy group having 2 to 10 carbon atoms, an alkynyloxy group having 2 to 10 carbon atoms, and a cycloalkenyloxy group having 3 to 10 carbon atoms, among the compounds represented by Formula (2)).

The present disclosure also relates to a compound represented by the following Formula (2D). 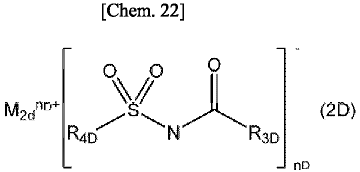 (In Formula (2D), R_{3D} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms. At least one hydrogen atom in the organic group is optionally substituted with a halogen atom. R_{4D} represents a fluorine atom. M_{2d}^{nD+} represents a proton, a metal cation, or an onium cation, and n_{D} represents a valence of the cation).

In Formula (2D), R_{3D} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms.

Specific examples in R_{3D} of an alkenyloxy group having 2 to 10 carbon atoms, an alkynyloxy group having 2 to 10 carbon atoms, and a cycloalkenyloxy group having 3 to 10 carbon atoms include those examples described above in R₃ for the alkenyloxy group having 2 to 10 carbon atoms, the alkynyloxy group having 2 to 10 carbon atoms, and the cycloalkenyloxy group having 3 to 10 carbon atoms.

At least one of any hydrogen atoms of the above organic group is optionally substituted with a halogen atom. Examples of the halogen atom include a fluorine atom, a chlorine atom, a bromine atom, and an iodine atom, a fluorine atom or a chlorine atom is preferable, and a fluorine atom is more preferable.

R_{3D} is preferably an alkenyloxy group having 2 to 3 carbon atoms or an alkynyloxy group having 2 to 3 carbon atoms, and more preferably an allyloxy group, or a 2-propynyloxy group.

In Formula (2D), M_{2d}^{nD+} represents a proton, a metal cation, or an onium cation. mo represents the valence of the cation.

When M_{2d}^{nD+} represents a metal cation, examples of the metal cation include an alkali metal cation such as a lithium ion, a sodium ion, and a potassium ion; an alkaline earth metal cation such as a magnesium ion and a calcium ion; or other metal cations such as a zinc ion.

When M_{2d}^{nD+} represents an onium cation, examples of the onium cation include a tetraalkylammonium ion.
m_{D} represents the valence of the cation. When M_{2d}^{nD+} represents a cation having a valence of 1, mo is 1, and when M_{2d}^{nD+} represents a cation having a valence of 2, m_{D} is 2.

M_{2d}^{nD+} preferably represents a metal cation, and more preferably represents a lithium ion or a sodium ion. When the nonaqueous electrolyte solution is used in a lithium ion battery, a lithium ion is more preferable, and when the nonaqueous electrolyte solution is used in a sodium ion battery, a sodium ion is more preferable.

Specific examples of the compound represented by the Formula (2D) include the specific examples of the compound represented by the Formula (2), that are included in the compound represented by Formula (2D) (specifically, a compound in which R₃ is an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and R₄ is a fluorine atom, among the specific examples of the compound represented by Formula (2)).

The compound represented by the Formula (1), (1A), (1B), (2), (2C) or (2D) is preferably used as an additive in the nonaqueous electrolyte solution.

The present disclosure also relates to an additive for a nonaqueous electrolyte solution containing at least one compound selected from the group consisting of the compound represented by the Formula (1) and the compound represented by the Formula (2).

### <(II) Solute>

The nonaqueous electrolyte solution according to the present disclosure preferably contains (II) a solute (hereinafter, also referred to as "component (II)").

The solute is not limited, but is preferably an ionic salt, and more preferably an ionic salt containing fluorine.

The solute is preferably, for example, an ionic salt containing a pair of at least one cation selected from the group consisting of alkali metal ions such as lithium ions and sodium ions, alkaline earth metal ions, and quaternary ammonium, and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, and a tris(trifluoromethanesulfonyl)methide anion.

The solute is preferably at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and Nal.

One type of these solutes may be used alone, or two or more types thereof may be mixed and used in any combination and any proportion according to an application.

Among the above solutes, in view of energy density, output characteristics, life, and the like of a nonaqueous electrolyte solution battery, the cation is preferably at least one selected from the group consisting of lithium, sodium, potassium, magnesium, and quaternary ammonium, and the anion is preferably at least one selected from the group consisting of a hexafluorophosphate anion, a tetrafluoroborate anion, a bis(trifluoromethanesulfonyl)imide anion, and a bis(fluorosulfonyl)imide anion.

A total amount of the solute in the nonaqueous electrolyte solution according to the present disclosure (hereinafter, also described as a "solute concentration") is not limited, but the solute concentration is preferably 0.5 mol/L or more, more preferably 0.7 mol/L or more, and further preferably 0.9 mol/L or more. Further, the solute concentration is preferably 5.0 mol/L or less, more preferably 4.0 mol/L or less, and further preferably 2.0 mol/L or less. Setting the solute concentration to 0.5 mol/L or more allows for reducing deterioration of the cycle characteristics and the output characteristics of the nonaqueous electrolyte solution battery due to deterioration in ionic conductivity, and setting the solute concentration to 5.0 mol/L or less allows for reducing the deterioration in ionic conductivity, and the deterioration in the cycle characteristics and the output characteristics of the nonaqueous electrolyte solution battery due to an increase in viscosity of the nonaqueous electrolyte solution.

### <(III) Nonaqueous Organic Solvent>

The nonaqueous electrolyte solution according to the present disclosure preferably contains a nonaqueous organic solvent (hereinafter, also referred to as "component (III)").

A type of the nonaqueous organic solvent that can be used in the nonaqueous electrolyte solution according to the present disclosure is not limited, and any nonaqueous organic solvent can be used.

The nonaqueous organic solvent is preferably at least one selected from the group consisting of a cyclic ester, a chain ester, a cyclic ether, a chain ether, a sulfone compound, a sulfoxide compound, and an ionic liquid.

Specifically, the nonaqueous organic solvent is preferably at least one selected from the group consisting of ethyl methyl carbonate (hereinafter, also described as "EMC"), dimethyl carbonate (hereinafter, also described as "DMC"), diethyl carbonate (hereinafter, also described as "DEC"), methyl propyl carbonate, ethyl propyl carbonate, methyl butyl carbonate, 2,2,2-trifluoroethyl methyl carbonate, 2,2,2-trifluoroethyl ethyl carbonate, 2,2,2-trifluoroethyl propyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter, also described as "EC"), propylene carbonate (hereinafter, also described as "PC"), butylene carbonate, fluoroethylene carbonate (hereinafter, also described as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethylsulfoxide, sulfolane, γ-butyrolactone, and γ-valerolactone.

In the present disclosure, an ionic liquid having a salt structure may be used as the nonaqueous organic solvent.

Preferably, the nonaqueous organic solvent contains at least one selected from the group consisting of a cyclic ester and a chain ester from a viewpoint of excellent input and output characteristics at low temperatures.

Further, preferably, the above nonaqueous organic solvent contains at least one selected from the group consisting of cyclic carbonates and chain carbonates from a viewpoint of excellent cycle characteristics at high temperatures.

Preferably, the nonaqueous organic solvent contains a cyclic ester, and the cyclic ester is a cyclic carbonate.

Specific examples of the above cyclic carbonate include EC, PC, butylene carbonate, and FEC, and at least one selected from the group consisting of EC, PC, and FEC is preferable.

Preferably, the nonaqueous organic solvent contains a chain ester, and the chain ester is chain carbonate.

Specific examples of the above chain carbonate include EMC, DMC, DEC, methyl propyl carbonate, ethyl propyl carbonate, 2,2,2-trifluoroethyl methyl carbonate, 2,2,2-trifluoroethyl ethyl carbonate, 1,1,1,3,3,3-hexafluoro-1-propylmethyl carbonate, and 1,1,1,3,3,3-hexafluoro-1-propylethyl carbonate, and at least one selected from the group consisting of EMC, DMC, DEC, and methyl propyl carbonate is preferable.

Specific examples of the above ester include methyl acetate, ethyl acetate, methyl propionate, ethyl propionate, methyl 2-fluoropropionate, ethyl 2-fluoropropionate, and methyl 3,3,3-trifluoropropionate.

### <Another Additive>

An additive component to be commonly used in the nonaqueous electrolyte solution according to the present disclosure may be further added in any proportion as long as the gist of the present disclosure is not impaired.

Specific examples of another additive include compounds having an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl-2-propynyl carbonate, ethyl-2-propynyl carbonate, di(2-propynyl) carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, 1,2-ethanedisulfonic anhydride, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, methanesulfonyl fluoride, ethenesulfonyl fluoride, and phenyl difluorophosphate.

The nonaqueous electrolyte solution according to the present disclosure may contain at least one selected from vinylene carbonate, bis(oxalato)borate, difluorooxalatoborate, difluorobis(oxalato)phosphate, tetrafluorooxalatophosphate, bis(fluorosulfonyl)imide salt, bis(difluorophosphoryl)imide salt, (difluorophosphoryl)(fluorosulfonyl)imide salt, difluorophosphate, fluorosulfonate, methanesulfonyl fluoride, 1,3,2-dioxathiolane-2,2-dioxide, 1,3-propenesultone, 1,3-propanesultone, 1,6-diisocyanatohexane, ethynylethylene carbonate, 4-propyl-1,3,2-dioxathiolane-2,2-dioxide, methylene methanedisulfonate, 1,2-ethanedisulfonic anhydride, tris(trimethylsilyl)borate, (ethoxy)pentafluorocyclotriphosphazene, tetrafluoro(malonato)phosphate, tetrafluoro(picolinato)phosphate, 1,3-dimethyl-1,3 -divinyl-1,3-di(1,1,1,3,3,3-hexafluoroisopropyl)disiloxane, tetravinylsilane, t-butylbenzene, t-amylbenzene, fluorobenzene, and cyclohexylbenzene. A content of the above additive in the nonaqueous electrolyte solution is preferably 0.01% by mass or more and 5.0% by mass or less with respect to the total amount of the nonaqueous electrolyte solution.

The nonaqueous electrolyte solution according to the present disclosure may contain a compound represented by the following Formula (4) as another additive.

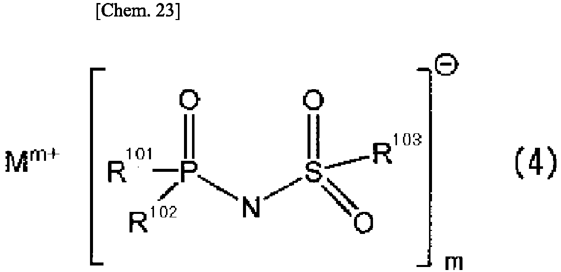

[In Formula (4), R¹⁰¹ to R¹⁰³ are each independently a fluorine atom or an organic group selected from a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group optionally has a fluorine atom, an oxygen atom, and/or an unsaturated bond. Here, at least one of R¹⁰¹ to R¹⁰³ is a fluorine atom. M^{m+} represents an alkali metal cation, an alkaline earth metal cation, or an onium cation, and m represents the same integer as a valence of the corresponding cation.]

When the compound (salt having an imide anion) represented by Formula (4) has at least one P-F bond or S-F bond, the compound achieves excellent low-temperature characteristics. The larger number of P-F bonds and S-F bonds in the salt having the above imide anion equates to the more improvement in low-temperature characteristics, which is preferable, and in the salt having the imide anion represented by the above Formula (4), a compound in which all of R¹⁰¹ to R¹⁰³ are fluorine atoms is more preferable.

In the salt having the imide anion represented by the above Formula (4), the preferable compound is a compound in which at least one of R¹⁰¹ to R¹⁰³ is a fluorine atom, and at least one of R¹⁰¹ to R¹⁰³ is selected from a hydrocarbon group having 6 or less carbon atoms and optionally having a fluorine atom.

In the salt having the imide anion represented by the above Formula (4), the preferable compound is a compound in which at least one of R¹⁰¹ to R¹⁰³ is a fluorine atom, and at least one of R¹⁰¹ to R¹⁰³ is selected from a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

A counter cation M^{m+} of the salt having the imide anion represented by the Formula (4) is preferably selected from the group consisting of lithium ion, sodium ion, potassium ion, and tetraalkylammonium ion.

In the Formula (4), examples of the alkyl group and the alkoxy group represented by R¹⁰¹ to R¹⁰³ include alkyl groups having 1 to 10 carbon atoms such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group, fluorine-containing alkyl groups, and alkoxy groups derived from these groups.

Examples of the alkenyl group and the alkenyloxy group include alkenyl groups having 2 to 10 carbon atoms such as a vinyl group, an allyl group, a 1-propenyl group, an isopropenyl group, a 2-butenyl group, and a 1,3-butadienyl group, fluorine-containing alkenyl groups, and alkenyloxy groups derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, fluorine-containing alkynyl groups, and alkynyloxy groups derived from these groups.

Examples of the cycloalkyl group and the cycloalkoxy group include a cycloalkyl group having 3 to 10 carbon atoms such as a cyclopentyl group and a cyclohexyl group, fluorine-containing cycloalkyl groups, and cycloalkoxy groups derived from these groups.

Examples of the cycloalkenyl group and the cycloalkenyloxy group include a cycloalkenyl group having 3 to 10 carbon atoms such as a cyclopentenyl group and a cyclohexenyl group, fluorine-containing cycloalkenyl groups, and cycloalkenyloxy groups derived from these groups.

Examples of the aryl group and the aryloxy group include an aryl group having 6 to 10 carbon atoms such as a phenyl group, a tolyl group, and an xylyl group, fluorine-containing aryl groups, and aryloxy groups derived from these groups.

Specific examples and synthesis methods of the salt having the imide anion represented by the Formula (4) include those described in WO2017/111143.

The content of another additive in the nonaqueous electrolyte solution is preferably 0.01% by mass or more and 8.0% by mass or less with respect to the total amount of the nonaqueous electrolyte solution.

When a content of the ionic salt exemplified as the solute is less than 0.5 mol/L, which is the lower limit of the suitable concentration of the solute, in the nonaqueous electrolyte solution, the ionic salt can exert a negative electrode film-forming effect and a positive electrode protective effect as "another additive". In this case, the content in the nonaqueous electrolyte solution is preferably 0.01% by mass to 5.0% by mass.

For example, when the nonaqueous electrolyte solution battery is a lithium ion battery, examples of the ionic salt in this case include lithium hexafluorophosphate, lithium tetrafluoroborate, lithium trifluoromethanesulfonate, lithium bis(trifluoromethanesulfonyl)imide, lithium bis(fluorosulfonyl)imide, and lithium (trifluoromethanesulfonyl)(fluorosulfonyl)imide, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, and sodium (trifluoromethanesulfonyl)(fluorosulfonyl)imide.

An alkali metal salt other than the above solutes may be used as an additive.

Specific examples include carboxylates such as lithium acrylate, sodium acrylate, lithium methacrylate, and sodium methacrylate, and sulfate ester salts such as lithium methyl sulfate, sodium methyl sulfate, lithium ethyl sulfate, and sodium ethyl sulfate.

From a viewpoint of improving durability (life) of a battery, when the nonaqueous electrolyte solution battery is a lithium ion battery, the nonaqueous electrolyte solution according to the present disclosure preferably contains, with respect to the total amount of the nonaqueous electrolyte solution, 0.01% by mass to 5.0% by mass of at least one selected from vinylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium bis(difluorophosphoryl)imide, lithium (difluorophosphoryl)(fluorosulfonyl)imide, lithium difluorophosphate, lithium fluorosulfonate, methanesulfonyl fluoride, tetravinylsilane, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide in the above another additive. In particular, the nonaqueous electrolyte solution preferably contains at least one selected from vinylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium bis(difluorophosphoryl)imide, lithium (difluorophosphoryl)(fluorosulfonyl)imide, lithium difluorophosphate, lithium fluorosulfonate, methanesulfonyl fluoride, tetravinylsilane, and 1,3,2-dioxathiolane-2,2-dioxide.

When the nonaqueous electrolyte solution battery is a sodium ion battery, the nonaqueous electrolyte solution preferably contains, with respect to the total amount of the nonaqueous electrolyte solution, 0.01% by mass to 5.0% by mass of at least one selected from vinylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium bis(difluorophosphoryl)imide, sodium (difluorophosphoryl)(fluorosulfonyl)imide, sodium difluorophosphate, sodium fluorosulfonate, methanesulfonyl fluoride, tetravinylsilane, 1,3-propenesultone, 1,3-propanesultone, 1,3,2-dioxathiolane-2,2-dioxide, 1,2-ethanedisulfonic anhydride, and 4-propyl-1,3,2-dioxathiolane-2,2-dioxide.

In addition, the nonaqueous electrolyte solution according to the present disclosure may also contain a polymer, and as in the case of being used in a nonaqueous electrolyte solution battery referred to as a polymer battery, the nonaqueous electrolyte solution can be quasi-solidified with a gelling agent or a cross-linked polymer. The polymer solid electrolyte includes one containing the nonaqueous organic solvent as a plasticizer.

The above polymer is not limited as long as the polymer is an aprotic polymer capable of dissolving the compound represented by the above Formula (1) or (2), the above solute, and the above another additive. Examples of the polymer include polymers having polyethylene oxide as a main chain or a side chain, homopolymers or copolymers of polyvinylidene fluoride, methacrylic acid ester polymers, and polyacrylonitrile. When a plasticizer is added to these polymers, an aprotic nonaqueous organic solvent is preferred among the above nonaqueous organic solvents.

### [2. Nonaqueous Electrolyte Solution Battery]

The nonaqueous electrolyte solution battery according to the present disclosure includes, at least, the nonaqueous electrolyte solution according to the present disclosure described above, a negative electrode, and a positive electrode. Furthermore, a separator, an exterior body, and the like are preferably included.

The negative electrode is not limited, but preferably uses a material capable of reversibly intercalating and deintercalating alkali metal ions such as lithium ions and sodium ions, or alkaline earth metal ions.

For example, in the case of the lithium ion secondary battery in which cations are mainly lithium, a negative electrode active material constituting the negative electrode is capable of doping and dedoping lithium ions. Examples of the negative electrode active material includes a material containing at least one selected from, for example, a carbon material in which a d value of a lattice plane (002 plane) in X-ray diffraction is 0.340 nm or less, a carbon material in which the d value of the lattice plane (002 plane) in X-ray diffraction exceeds 0.340 nm, oxides of one or more metal selected from Si, Sn, and Al, one or more metals selected from Si, Sn, and Al, alloys containing these metals, alloys of these metals and lithium, or alloys of the above alloys and lithium, and lithium titanium oxide. One of these negative electrode active materials can be used alone, or two or more thereof can be used in combination. Lithium metal, metal nitrides, tin compounds, conductive polymers, and the like may also be used.

For example, in the case of a sodium ion secondary battery in which a cation is mainly sodium, as the negative electrode active material constituting the negative electrode, sodium metal, an alloy of sodium metal and other metals such as tin, an intermetallic compound, various carbon materials such as hard carbon, a metal oxide such as titanium oxide, a metal nitride, (elemental) tin, a tin compound, activated carbon, a conductive polymer, and the like are used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like are used. One type of these negative electrode active materials may be used alone, or two or more types thereof may be used in combination.

The positive electrode is not limited, but preferably uses a material capable of reversibly intercalating and deintercalating alkali metal ions such as lithium ions and sodium ions, or alkaline earth metal ions.

For example, when the cation is lithium, lithium-containing transition metal composite oxides such as LiCoO₂, LiNiO₂, LiMnO₂, and LiMn₂O₄, these lithium-containing transition metal composite oxides including a mixture of a plurality of transition metals such as Co, Mn, and Ni, these lithium-containing transition metal composite oxides in which a part of the transition metals is substituted with a metal other than the transition metals, phosphate compounds of transition metals such as LiFePO₄, LiCoPO₄, and LiMnPO₄ referred to as olivine, oxides such as TiO₂, V₂O₅, and MoOs, sulfides such as TiS₂ and FeS, conductive polymers such as polyacetylene, polyparaphenylene, polyaniline, and polypyrrole, activated carbon, radical-generating polymers, carbon materials, and the like can be used as a positive electrode material.

For example, when the cation is sodium, sodium-containing transition metal composite oxides such as NaCrO₂, NaFe_{0.5}Co_{0.5}O₂, NaFe_{0.4}Mn_{0.3}Ni_{0.3}O₂, NaNi_{0.5}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Tii_{1/6}Mn_{1/2}O₂, and Na_{2/3}Ni_{1/3}Mn_{2/3}O₂, these sodium-containing transition metal composite oxides including a mixture of a plurality of transition metals such as Co, Mn, and Ni, these sodium-containing transition metal composite oxides in which a part of the transition metals is substituted with a metal other than the transition metals, polyanion type compounds such as NaFePO₄, NaVPO₄F, Na₃V₂(PO₄)₃, and Na₂Fe₂(SO₄)₃, sodium salts of Prussian Blue analogues represented by a composition formula NaₐM_{b}[Fe(CN)₆]_{c} (M = Cr, Mn, Fe, Co, Ni, Cu, or Zn, 0 ≤ a ≤ 2, 0.5 ≤ b ≤ 1.5, 0.5 ≤ c ≤ 1.5), oxides such as TiO₂, V₂O₅, and MoOs, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material (positive electrode active material).

An electrode sheet obtained by mixing acetylene black, ketjen black, carbon fiber, or graphite as a conductive material, and polytetrafluoroethylene, polyvinylidene fluoride, SBR resin, or the like as a binder and molding the mixture into a sheet shape can be used to the positive electrode material and negative electrode material.

As a separator for preventing contact between the positive electrode and the negative electrode, a nonwoven fabric or porous sheet made of polyolefin, polyethylene, paper, glass fiber, or the like may be used.

An electrochemical device having a shape such as a coin shape, a cylindrical shape, a square shape, or an aluminum laminate sheet shape is assembled based on the above elements.

The present disclosure also relates to a method for producing a nonaqueous electrolyte solution battery, and the method includes: a preparing the nonaqueous electrolyte solution; and filling an empty cell including a positive electrode and a negative electrode with the nonaqueous electrolyte solution.

### Examples

Hereinafter, the present disclosure will be described in more detail below with reference to Examples, but the present disclosure is not limited by these descriptions.

### <Synthesis Example 1>

### Synthesis of Compound (1a)

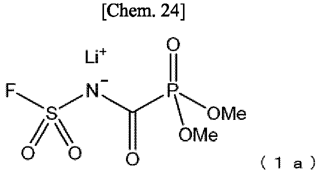

After 30 g of acetonitrile (MeCN) and 1.1 g of dimethyl phosphite were put into a 50 ml eggplant flask, 1.4 g of chlorosulfonyl isocyanate was slowly added. After an internal temperature of the flask lowered to a room temperature (25°C), 0.5 g of sodium fluoride was added. The reaction solution was filtered, and 0.08 g of lithium hydride was added to the filtrate. After foaming ceased, concentration was carried out to obtain 2.0 g of a compound (1a) (yield: 83%).

¹H NMR(CD₃CN) σ_{H} 3.79 ppm, ¹⁹F NMR(CD₃CN) σ_{F} 50.5 ppm.

In the above structural formula, Me represents a methyl group.

### <Synthesis Example 2>

### Synthesis of Compound (2a)

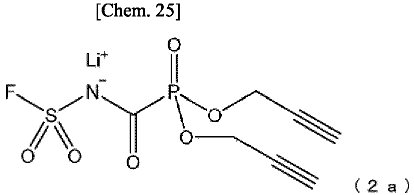

After 30 g of acetonitrile (MeCN) and 1.6 g of di(2-propynyl) phosphite were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 2.3 g of a compound (2a) (yield: 80%).

¹H NMR(CD₃CN) σ_{H} 4.78 ppm, 2.92 ppm, ¹⁹F NMR(CD₃CN) σ_{F} 50.3 ppm.

### <Synthesis Example 3>

### Synthesis of Compound (3a)

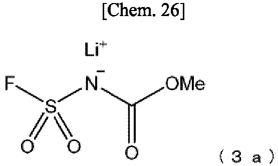

After 30 g of acetonitrile (MeCN) and 0.3 g of methanol were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 1.5 g of a compound (3a) (yield: 92%).

¹H NMR(CD₃CN) σ_{H} 3.67 ppm, ¹⁹F NMR(CD₃CN) σ_{F} 51.1 ppm.

In the above structural formula, Me represents a methyl group.

### <Synthesis Example 4>

### Synthesis of Compound (4a)

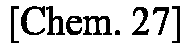

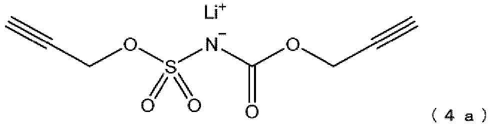

After 30 g of acetonitrile (MeCN) and 1.2 g of 2-propynyl alcohol were put into a 50 ml eggplant flask, 1.4 g of chlorosulfonyl isocyanate was slowly added. After an internal temperature lowered to a room temperature, 0.16 g of lithium hydride was added. The reaction solution was filtered, and concentration was carried out to obtain 2.0 g of a compound (4a) (yield: 90%).

¹H NMR (CD₃CN) on 4.61 ppm, 4.57 ppm, 2.82 ppm, and 2.70 ppm.

### <Synthesis Example 5>

### Synthesis of Compound (5a)

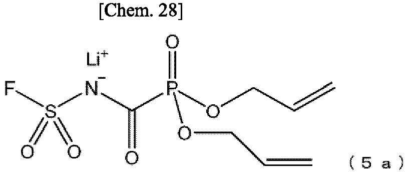

After 30 g of acetonitrile (MeCN) and 1.6 g of diallyl phosphite were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 2.5 g of a compound (5a) (yield: 85%).

¹H NMR(CD₃CN) σ_{H} 5.96 ppm, 5.36 ppm, 5.23 ppm, and 4.61 ppm, ¹⁹F NMR(CD₃CN) σ_{F} 50.2 ppm.

### <Synthesis Example 6>

### Synthesis of Compound (6a)

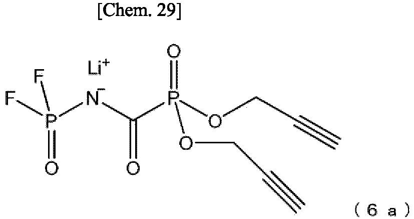

After 30 g of acetonitrile (MeCN) and 1.6 g of di-2-propynyl phosphite were put into a 50 ml eggplant flask, 1.6 g of dichlorophosphoryl isocyanate was slowly added. After an internal temperature of the flask lowered to a room temperature, 1.0 g of sodium fluoride was added. The reaction solution was filtered, and 0.08 g of lithium hydride was added to the filtrate. After foaming ceased, concentration was carried out to obtain 1.5 g of a compound (6a) (yield: 52%).

¹H NMR(CD₃CN) σ_{H} 4.59 ppm, 2.81 ppm, ¹⁹F NMR(CD₃CN) σ_{F} 77.2 ppm.

### <Synthesis Example 7>

### Synthesis of Compound (7a)

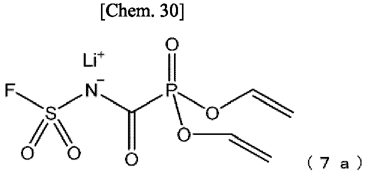

After 30 g of acetonitrile (MeCN) and 1.3 g of divinyl phosphite were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 1.9 g of a compound (7a) (yield: 72%).

¹H NMR(CD₃CN) σ_{H} 4.78 ppm, 2.92 ppm, ¹⁹F NMR(CD₃CN) σ_{F} 50.3 ppm.

### <Synthesis Example 8>

### Synthesis of Compound (8a)

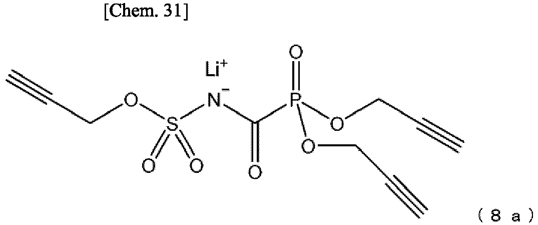

After 30 g of acetonitrile (MeCN) and 1.6 g of di-2-propynyl phosphite were put into a 50 ml eggplant flask, 1.4 g of chlorosulfonyl isocyanate was slowly added. After an internal temperature lowered to a room temperature, 0.6 g of 2-propynyl alcohol and 0.16 g of lithium hydride were added. After foaming ceased, the reaction solution was filtered, and concentration of the filtrate was carried out to obtain 2.4 g of a compound (8a) (yield: 74%).

¹H NMR (CD₃CN) σ_{H} 4.82 ppm, 4.75 ppm, 2.99 ppm, and 2.91 ppm.

### <Synthesis Example 9>

### Synthesis of Compound (9a)

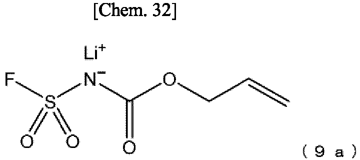

After 30 g of acetonitrile (MeCN) and 0.6 g of allyl alcohol were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 1.7 g of a compound (9a) (yield: 90%).

¹H NMR(CD₃CN) σ_{H} 5.91 ppm, 5.26 ppm, 5.17 ppm, and 4.48 ppm, ¹⁹F NMR(CD₃CN) σ_{F} 49.7 ppm.

### <Synthesis Example 10>

### Synthesis of Compound (10a)

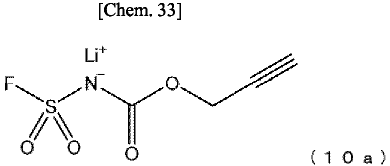

After 30 g of acetonitrile (MeCN) and 0.6 g of 2-propynyl alcohol were put into a 50 ml eggplant flask, 1.4 g of chlorosulfonyl isocyanate was slowly added. After an internal temperature of the flask lowered to a room temperature, 0.5 g of sodium fluoride was added. The reaction solution was filtered, and 0.08 g of lithium hydride was added to the filtrate. After foaming ceased, concentration was carried out to obtain 1.7 g of a compound (10a) (yield: 90%).

¹H NMR(CD₃CN) σ_{H} 4.60 ppm, 2.71 ppm, ¹⁹F NMR(CD₃CN) σ_{F} 49.5 ppm.

### <Synthesis Example 11>

### Synthesis of Compound (1 la)

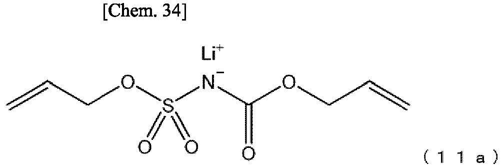

After 30 g of acetonitrile (MeCN) and 1.2 g of allyl alcohol were put into a 50 ml eggplant flask, 1.4 g of chlorosulfonyl isocyanate was slowly added. After an internal temperature lowered to a room temperature, 0.16 g of lithium hydride was added. After foaming ceased, the reaction solution was filtered, and concentration of the filtrate was carried out to obtain 2.1 g of a compound (11a) (yield: 93%).

¹H NMR (CD₃CN) on 5.93 ppm, 5.43 ppm, 5.33 ppm, 5.24 ppm, 4.75 ppm, and 4.62 ppm.

### <Synthesis Example 12>

### Synthesis of Compound (12a)

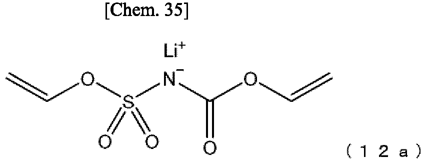

After 30 g of acetonitrile (MeCN) and 1.2 g of 2-chloroethanol were put into a 50 ml eggplant flask, 1.4 g of chlorosulfonyl isocyanate was slowly added. After an internal temperature lowered to a room temperature, 0.32 g of lithium hydride was added. After foaming ceased, the reaction solution was filtered, and concentration of the filtrate was carried out to obtain 2.0 g of a compound (12a) (yield: 90%).

¹H NMR (CD₃CN) σ_{H} 4.61 ppm, 4.57 ppm, 2.82 ppm, and 2.70 ppm.

### [Preparation of Nonaqueous Electrolyte Solutions according to Examples and Comparative Examples]

### <Comparative Example 1-1>

### (Preparation of LiPF₆ Solution)

EC, FEC, EMC, and DMC were mixed in a volume ratio of EC:FEC:EMC:DMC = 25:5:45:25 in a glove box having a dew point of -60°C or lower (component (III)). After that, LiPF₆ (component (II)) was added such that LiPF₆ had a concentration of 1.0 mol/L, with an internal temperature at 40°C or lower kept. The mixture was stirred, and LiPF₆ was completely dissolved to obtain a LiPF₆ solution. This LiPF₆ solution was set as a comparative nonaqueous electrolyte solution 1-1.

### <Example 1-1>

### (Preparation of Nonaqueous Electrolyte Solution 1-1)

EC, FEC, EMC, and DMC were mixed in a volume ratio of EC:FEC:EMC:DMC = 25:5:45:25 in a glove box having a dew point of -60°C or lower (component (III)). After that, LiPF₆ (component (II)) was added such that LiPF₆ had a concentration of 1.0 mol/L, with an internal temperature at 40°C or lower kept. The compound (1a) (component (I)) corresponding to the compound represented by Formula (1) was added such that the compound (1a) had a concentration of 0.05% by mass with respect to the total amount of the nonaqueous electrolyte solution. Then, the mixture was stirred and dissolved for 1 hour to obtain a nonaqueous electrolyte solution 1-1 according to Example 1-1.

### <Examples 1-2 to 1-12 and Comparative Examples 1-2 to 1-5>

### (Preparation of Nonaqueous Electrolyte Solutions 1-2 to 1-12 and Comparative Nonaqueous Electrolyte Solutions 1-2 to 1-5)

Except that the type and content of the component (I) (or a comparative compound) were changed as described in Table 1, nonaqueous electrolyte solutions 1-2 to 1-12 and comparative nonaqueous electrolyte solutions 1-2 to 1-5 were obtained in the same way as the preparation of the nonaqueous electrolyte solution 1-1.

Structures of a compound used in Comparative Examples are shown below. In the following structural formula, Et represents an ethyl group, and Bu represents a butyl group.

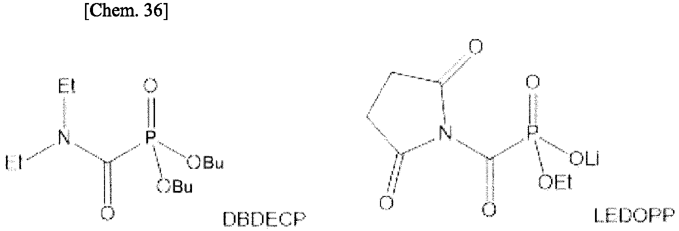

### <Examples 2-1 to 2-4 and Comparative Examples 2-1 to 2-3>

### (Preparation of Nonaqueous Electrolyte Solutions 2-1 to 2-4 and Comparative Nonaqueous Electrolyte Solutions 2-1 to 2-3)

Furthermore, except that vinylene carbonate (VC) as another additive was added and dissolved such that vinylene carbonate had a concentration described in Table 2, nonaqueous electrolyte solutions 2-1 to 2-4 and comparative nonaqueous electrolyte solutions 2-1 to 2-3 were respectively obtained in the same way as in the preparation of the nonaqueous electrolyte solutions 1-4, 1-8, 1-10, and 1-12 and the comparative nonaqueous electrolyte solutions 1-1, 1-3, and 1-5.

### <Examples 3-1 to 3-4 and Comparative Examples 3-1 to 3-3>

### (Preparation of Nonaqueous Electrolyte Solutions 3-1 to 3-4 and Comparative Nonaqueous Electrolyte Solutions 3-1 to 3-3)

Except that VC was changed to lithium bis(oxalato)borate (BOB), nonaqueous electrolyte solutions 3-1 to 3-4 and comparative nonaqueous electrolyte solutions 3-1 to 3-3 were respectively obtained in the same way as the preparation of the nonaqueous electrolyte solutions 2-1 to 2-4 and the comparative nonaqueous electrolyte solutions 2-1 to 2-3.

### <Examples 4-1 to 4-4 and Comparative Examples 4-1 to 4-3>

### (Preparation of Nonaqueous Electrolyte Solutions 4-1 to 4-4 and Comparative Nonaqueous Electrolyte Solutions 4-1 to 4-3)

Except that VC was changed to lithium difluorobis(oxalato)phosphate (DFBOP), nonaqueous electrolyte solutions 4-1 to 4-4 and comparative nonaqueous electrolyte solutions 4-1 to 4-3 were respectively obtained in the same way as the preparation of the nonaqueous electrolyte solutions 2-1 to 2-4 and the comparative nonaqueous electrolyte solutions 2-1 to 2-3.

### <Examples 5-1 to 5-4 and Comparative Examples 5-1 to 5-3>

### (Preparation of Nonaqueous Electrolyte Solutions 5-1 to 5-4 and Comparative Nonaqueous Electrolyte Solutions 5-1 to 5-3)

Except that VC was changed to lithium tetrafluorooxalatophosphate (TFOP), nonaqueous electrolyte solutions 5-1 to 5-4 and comparative nonaqueous electrolyte solutions 5-1 to 5-3 were respectively obtained in the same way as the preparation of the nonaqueous electrolyte solutions 2-1 to 2-4 and the comparative nonaqueous electrolyte solutions 2-1 to 2-3.

### <Examples 6-1 to 6-4 and Comparative Examples 6-1 to 6-3>

### (Preparation of Nonaqueous Electrolyte Solutions 6-1 to 6-4 and Comparative Nonaqueous Electrolyte Solutions 6-1 to 6-3)

Except that VC was changed to lithium bis(fluorosulfonyl)imide (FSI), nonaqueous electrolyte solutions 6-1 to 6-4 and comparative nonaqueous electrolyte solutions 6-1 to 6-3 were respectively obtained in the same way as the preparation of the nonaqueous electrolyte solutions 2-1 to 2-4 and the comparative nonaqueous electrolyte solutions 2-1 to 2-3.

### <Examples 7-1 to 7-4 and Comparative Examples 7-1 to 7-3>

### (Preparation of Nonaqueous Electrolyte Solutions 7-1 to 7-4 and Comparative Nonaqueous Electrolyte Solutions 7-1 to 7-3)

Except that VC was changed to lithium difluorophosphate (DFP), nonaqueous electrolyte solutions 7-1 to 7-4 and comparative nonaqueous electrolyte solutions 7-1 to 7-3 were respectively obtained in the same way as the preparation of the nonaqueous electrolyte solution 2-1 to 2-4 and the comparative nonaqueous electrolyte solutions 2-1 to 2-3.

### <Examples 8-1 to 8-12 and Comparative Examples 8-1 to 8-3>

### (Preparation of Nonaqueous Electrolyte Solutions 8-1 to 8-12 and Comparative Nonaqueous Electrolyte Solutions 8-1 to 8-3)

Except that VC was changed to lithium fluorosulfonate (FS), and a type of the component (I) (or the comparative compound) was changed as described in Table 8, nonaqueous electrolyte solutions 8-1 to 8-12 and comparative nonaqueous electrolyte solutions 8-1 to 8-3 were respectively obtained in the same way as in the preparation of the nonaqueous electrolyte solutions 2-1 to 2-4 and the comparative nonaqueous electrolyte solutions 2-1 to 2-3.

In the following Tables 1 to 16, VC means vinylene carbonate, BOB means lithium bis(oxalato)borate, DFBOP means lithium difluorobis(oxalato)phosphate, TFOP means lithium tetrafluorooxalatophosphate, FSI means lithium bis(fluorosulfonyl)imide, DFP means lithium difluorophosphate, and FS means lithium fluorosulfonate.

In the following Tables 1 to 16, the content of the component (1) (or the comparative compound) represents the concentration with respect to the total amount of nonaqueous electrolyte solution. The content of another additive represents the concentration with respect to the total amount of the nonaqueous electrolyte solution.

### [Production of Nonaqueous Electrolyte Solution Battery]

### (Production of NCM622 Positive Electrode)

5% by mass of polyvinylidene fluoride (hereinafter, also described as PVDF) as a binder and 5% by mass of acetylene black as a conductive material were mixed to 90% by mass of LiNi_{0.6}Co_{0.2}Mn_{0.2}O₂ powder, and N-methyl-2-pyrrolidone was further added to produce a positive electrode mixture paste. The paste was applied onto both sides of aluminum foil (A1085), and the resultant product was dried and pressed, and then punched into 4 cm × 5 cm to obtain a test NCM622 positive electrode.

### (Production of NCM811 Positive Electrode)

3.5% by mass of PVDF as a binder and 4.5% by mass of acetylene black as a conductive material were mixed to 92.0% by mass of LiNi_{0.8}Co_{0.1}Mn_{0.1}O₂ powder, and N-methyl-2-pyrrolidone was further added to produce a positive electrode mixture paste. The paste was applied onto both sides of aluminum foil (A1085), and the resultant product was dried and pressed, and then punched into 4 cm × 5 cm to obtain an NCM811 positive electrode for test.

### (Production of Natural Graphite Negative Electrode)

90% by mass of natural graphite powder, 5% by mass of PVDF as a binder, and 5% by mass of acetylene black as a conductive material were mixed to produce a negative electrode mixture paste. The paste was applied onto one side of copper foil, dried and pressed, and then punched into 4 cm × 5 cm to obtain a natural graphite negative electrode for a test.

### (Production of Silicon-containing Graphite Negative Electrode)

To 85% by mass of artificial graphite powder, 7% by mass of nanosilicon, 3% by mass of conductive material (HS-100), 2% by mass of carbon nanofiber (VGCF), 2% by mass of styrene-butadiene rubber, 1% by mass of sodium carboxymethylcellulose, and water were mixed, and a negative electrode mixture paste was produced. The paste was applied onto one side of copper foil, and the resultant product was dried and pressed, and then punched into 4 cm × 5 cm to obtain a silicon-containing graphite negative electrode for a test.

### (Production of Nonaqueous Electrolyte Solution Battery)

Under an argon atmosphere at a dew point of -50°C or lower, a terminal was welded to the above NCM622 positive electrode, and both sides of the welded product were then stacked between two polyethylene separators (5 cm × 6 cm), and the outside of the stacked product were stacked between two natural graphite negative electrodes to which a terminal had been welded in advance so that a surface of the negative electrode active material faces a surface of the positive electrode active material. The resultant product was put in an aluminum laminated bag having an opening on one side, the nonaqueous electrolyte solution was vacuum-injected into the bag, and the opening was then sealed with heat to produce aluminum laminated nonaqueous electrolyte solution batteries according to Examples and Comparative Examples. The nonaqueous electrolyte solution used those described in Tables 1 to 8.

Further, in Examples 9-1 to 9-12, Comparative Examples 9-1 to 9-5, Examples 10-1 to 10-4, Comparative Examples 10-1 to 10-3, Examples 11-1 to 11-4, Comparative Examples 11-1 to 11-3, Examples 12-1 to 12-4, Comparative Examples 12-1 to 12-3, Examples 13-1 to 13-4, Comparative Examples 13-1 to 13-3, Examples 14-1 to 14-4, Comparative Examples 14-1 to 14-3, Examples 15-1 to 15-4, Comparative Examples 15-1 to 15-3, Examples 16-1 to 16-12, and Comparative Examples 16-1 to 16-3, NCM811 as the positive electrode and silicon-containing graphite as the negative electrode were used to produce a nonaqueous electrolyte solution battery in the same manner. The nonaqueous electrolyte solution used those described in Tables 9 to 16.

### [Evaluation]

### -Initial Charge and Discharge-

The produced nonaqueous electrolyte solution battery was put in a 25°C constant temperature bath and, in this state, connected to a charge and discharge device. Charge was performed at 3 mA until 4.3 V. After 4.3 V was maintained for one hour, discharge was performed at 6 mA to 2.5 V. This was defined as one charge and discharge cycle, and three cycles in total of charge and discharge were performed to stabilize the battery.

### [Initial Resistance Measurement]

The test cell described above was charged to 4.3 V at 25°C and 6 mA, and then a resistance value was measured by impedance measurement in a -20°C environment.

### [Evaluation of High-temperature Cycle Characteristic]

The test cell was subjected to a charge and discharge test at an environmental temperature of 60°C to evaluate a cycle characteristic. The charge and discharge cycle was repeated at a current value of 30 mA by using a constant current constant voltage method with an upper limit charge voltage of 4.3 V and a lower limit discharge voltage of 2.5 V. Further, a degree of deterioration of the cell was evaluated with the discharge capacity maintenance rate at a 500th cycle in the charge and discharge test at the environmental temperature of 60°C. A "discharge capacity maintenance rate after a high-temperature cycle" represented by the discharge capacity maintenance rate at the 500th cycle was obtained by the following formula. A discharge capacity at a first cycle in the charge and discharge test at the environmental temperature of 60°C was defined as an initial discharge capacity. Discharge capacity maintenance rate after high-temperature cycle (%) = (discharge capacity at 500th cycle/initial discharge capacity) × 100

In each of Tables 1 to 16, the initial resistance value and the discharge capacity maintenance rate after a high-temperature cycle of Comparative Examples using the comparative nonaqueous electrolyte solution to which neither the component (I) nor a comparative compound had been added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8, Comparative Example 9-1 in Table 9, Comparative Example 10-1 in Table 10, Comparative Example 11-1 in Table 11, Comparative Example 12-1 in Table 12, Comparative Example 13-1 in Table 13, Comparative Example 14-1 in Table 14, Comparative Example 15-1 in Table 15, and Comparative Example 16-1 in Table 16) were respectively defined as 100, and evaluation results in each of Examples and Comparative Examples were shown as relative values.

**[Table 1]**

| Table 1 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 1-1 | Nonaqueous electrolyte solution 1-1 | (1a) | 0.05 | - | - | 92.1 | 101.1 |
| Example 1-2 | Nonaqueous electrolyte solution 1-2 | (1a) | 0.1 | - | - | 85.2 | 101.3 |
| Example 1-3 | Nonaqueous electrolyte solution 1-3 | (1a) | 0.5 | - | - | 75.2 | 103.1 |
| Example 1-4 | Nonaqueous electrolyte solution 1-4 | (1a) | 1.0 | - | - | 75.0 | 104.2 |
| Example 1-5 | Nonaqueous electrolyte solution 1-5 | (1a) | 2.0 | - | - | 81.5 | 104.5 |
| Example 1-6 | Nonaqueous electrolyte solution 1-6 | (1a) | 5.0 | - | - | 90.9 | 104.6 |
| Example 1-7 | Nonaqueous electrolyte solution 1-7 | (2a) | 0.5 | - | - | 77.0 | 104.5 |
| Example 1-8 | Nonaqueous electrolyte solution 1-8 | (2a) | 1.0 | - | - | 77.8 | 105.8 |
| Example 1-9 | Nonaqueous electrolyte solution 1-9 | (3a) | 0.5 | - | - | 73.5 | 100.8 |
| Example 1-10 | Nonaqueous electrolyte solution 1-10 | (3a) | 1.0 | - | - | 70.1 | 102.1 |
| Example 1-11 | Nonaqueous electrolyte solution 1-11 | (4a) | 0.5 | - | - | 78.9 | 103.6 |
| Example 1-12 | Nonaqueous electrolyte solution 1-12 | (4a) | 1.0 | - | - | 78.1 | 106.2 |
| Comparative Example 1-1 | Comparative nonaqueous electrolyte solution 1-1 | - | - | - | - | 100 | 100 |
| Comparative Example 1-2 | Comparative nonaqueous electrolyte solution 1-2 | DBDECP | 0.5 | - | - | 117.3 | 98.5 |
| Comparative Example 1-3 | Comparative nonaqueous electrolyte solution 1-3 | DBDECP | 1.0 | - | - | 130.5 | 95.3 |
| Comparative Example 1-4 | Comparative nonaqueous electrolyte solution 1-4 | LEDOPP | 0.5 | - | - | 100.7 | 100.5 |
| Comparative Example 1-5 | Comparative nonaqueous electrolyte solution 1-5 | LEDOPP | 1.0 | - | - | 103.1 | 102.1 |

**[Table 2]**

| Table 2 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 2-1 | Nonaqueous electrolyte solution 2-1 | (1a) | 1.0 | VC | 1.0 | 76.5 | 105.2 |
| Example 2-2 | Nonaqueous electrolyte solution 2-2 | (2a) | 1.0 | VC | 1.0 | 73.1 | 107.8 |
| Example 2-3 | Nonaqueous electrolyte solution 2-3 | (3a) | 1.0 | VC | 1.0 | 69.2 | 103.4 |
| Example 2-4 | Nonaqueous electrolyte solution 2-4 | (4a) | 1.0 | VC | 1.0 | 72.1 | 107.7 |
| Comparative Example 2-1 | Comparative nonaqueous electrolyte solution 2-1 | - | - | VC | 1.0 | 100 | 100 |
| Comparative Example 2-2 | Comparative nonaqueous electrolyte solution 2-2 | DBDECP | 1.0 | VC | 1.0 | 112.5 | 95.2 |
| Comparative Example 2-3 | Comparative nonaqueous electrolyte solution 2-3 | LEDOPP | 1.0 | VC | 1.0 | 101.1 | 100.1 |

**[Table 3]**

| Table 3 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 3-1 | Nonaqueous electrolyte solution 3-1 | (1a) | 1.0 | BOB | 1.0 | 72.1 | 105.9 |
| Example 3-2 | Nonaqueous electrolyte solution 3-2 | (2a) | 1.0 | BOB | 1.0 | 69.5 | 105.5 |
| Example 3-3 | Nonaqueous electrolyte solution 3-3 | (3a) | 1.0 | BOB | 1.0 | 67.1 | 102.1 |
| Example 3-4 | Nonaqueous electrolyte solution 3-4 | (4a) | 1.0 | BOB | 1.0 | 70.2 | 108.2 |
| Comparative Example 3-1 | Comparative nonaqueous electrolyte solution 3-1 | - | - | BOB | 1.0 | 100 | 100 |
| Comparative Example 3-2 | Comparative nonaqueous electrolyte solution 3-2 | DBDECP | 1.0 | BOB | 1.0 | 120.1 | 93.7 |
| Comparative Example 3-3 | Comparative nonaqueous electrolyte solution 3-3 | LEDOPP | 1.0 | BOB | 1.0 | 99.5 | 99.8 |

**[Table 4]**

| Table 4 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 4-1 | Nonaqueous electrolyte solution 4-1 | (1a) | 1.0 | DFBOP | 1.0 | 65.3 | 105.5 |
| Example 4-2 | Nonaqueous electrolyte solution 4-2 | (2a) | 1.0 | DFBOP | 1.0 | 64.1 | 106.8 |
| Example 4-3 | Nonaqueous electrolyte solution 4-3 | (3a) | 1.0 | DFBOP | 1.0 | 65.1 | 102.3 |
| Example 4-4 | Nonaqueous electrolyte solution 4-4 | (4a) | 1.0 | DFBOP | 1.0 | 67.1 | 109.8 |
| Comparative Example 4-1 | Comparative nonaqueous electrolyte solution 4-1 | - | - | DFBOP | 1.0 | 100 | 100 |
| Comparative Example 4-2 | Comparative nonaqueous electrolyte solution 4-2 | DBDECP | 1.0 | DFBOP | 1.0 | 121.2 | 92.1 |
| Comparative Example 4-3 | Comparative nonaqueous electrolyte solution 4-3 | LEDOPP | 1.0 | DFBOP | 1.0 | 101.3 | 98.1 |

**[Table 5]**

| Table 5 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 5-1 | Nonaqueous electrolyte solution 5-1 | (1a) | 1.0 | TFOP | 1.0 | 68.1 | 103.5 |
| Example 5-2 | Nonaqueous electrolyte solution 5-2 | (2a) | 1.0 | TFOP | 1.0 | 65.0 | 105.8 |
| Example 5-3 | Nonaqueous electrolyte solution 5-3 | (3a) | 1.0 | TFOP | 1.0 | 63.8 | 102.6 |
| Example 5-4 | Nonaqueous electrolyte solution 5-4 | (4a) | 1.0 | TFOP | 1.0 | 65.0 | 106.4 |
| Comparative Example 5-1 | Comparative nonaqueous electrolyte solution 5-1 | - | - | TFOP | 1.0 | 100 | 100 |
| Comparative Example 5-2 | Comparative nonaqueous electrolyte solution 5-2 | DBDECP | 1.0 | TFOP | 1.0 | 120.2 | 93.8 |
| Comparative Example 5-3 | Comparative nonaqueous electrolyte solution 5-3 | LEDOPP | 1.0 | TFOP | 1.0 | 102.2 | 99.9 |

**[Table 6]**

| Table 6 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 6-1 | Nonaqueous electrolyte solution 6-1 | (1a) | 1.0 | FSI | 1.0 | 72.3 | 104.4 |
| Example 6-2 | Nonaqueous electrolyte solution 6-2 | (2a) | 1.0 | FSI | 1.0 | 70.5 | 106.3 |
| Example 6-3 | Nonaqueous electrolyte solution 6-3 | (3a) | 1.0 | FSI | 1.0 | 68.2 | 102.2 |
| Example 6-4 | Nonaqueous electrolyte solution 6-4 | (4a) | 1.0 | FSI | 1.0 | 71.0 | 108.8 |
| Comparative Example 6-1 | Comparative nonaqueous electrolyte solution 6-1 | - | - | FSI | 1.0 | 100 | 100 |
| Comparative Example 6-2 | Comparative nonaqueous electrolyte solution 6-2 | DBDECP | 1.0 | FSI | 1.0 | 121.3 | 94.1 |
| Comparative Example 6-3 | Comparative nonaqueous electrolyte solution 6-3 | LEDOPP | 1.0 | FSI | 1.0 | 100.0 | 100.9 |

**[Table 7]**

| Table 7 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 7-1 | Nonaqueous electrolyte solution 7-1 | (1a) | 1.0 | DFP | 1.0 | 65.5 | 105.1 |
| Example 7-2 | Nonaqueous electrolyte solution 7-2 | (2a) | 1.0 | DFP | 1.0 | 64.7 | 105.8 |
| Example 7-3 | Nonaqueous electrolyte solution 7-3 | (3a) | 1.0 | DFP | 1.0 | 65.0 | 105.3 |
| Example 7-4 | Nonaqueous electrolyte solution 7-4 | (4a) | 1.0 | DFP | 1.0 | 66.7 | 106.6 |
| Comparative Example 7-1 | Comparative nonaqueous electrolyte solution 7-1 | - | - | DFP | 1.0 | 100 | 100 |
| Comparative Example 7-2 | Comparative nonaqueous electrolyte solution 7-2 | DBDECP | 1.0 | DFP | 1.0 | 124.4 | 96.9 |
| Comparative Example 7-3 | Comparative nonaqueous electrolyte solution 7-3 | LEDOPP | 1.0 | DFP | 1.0 | 102.8 | 101.8 |

**[Table 8]**

| Table 8 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 8-1 | Nonaqueous electrolyte solution 8-1 | (1a) | 1.0 | FS | 1.0 | 76.5 | 104.9 |
| Example 8-2 | Nonaqueous electrolyte solution 8-2 | (2a) | 1.0 | FS | 1.0 | 74.1 | 108.8 |
| Example 8-3 | Nonaqueous electrolyte solution 8-3 | (3a) | 1.0 | FS | 1.0 | 72.0 | 103.2 |
| Example 8-4 | Nonaqueous electrolyte solution 8-4 | (4a) | 1.0 | FS | 1.0 | 73.9 | 109.5 |
| Example 8-5 | Nonaqueous electrolyte solution 8-5 | (5a) | 1.0 | FS | 1.0 | 80.3 | 110.7 |
| Example 8-6 | Nonaqueous electrolyte solution 8-6 | (6a) | 1.0 | FS | 1.0 | 83.5 | 102.5 |
| Example 8-7 | Nonaqueous electrolyte solution 8-7 | (7a) | 1.0 | FS | 1.0 | 78.1 | 107.8 |
| Example 8-8 | Nonaqueous electrolyte solution 8-8 | (8a) | 1.0 | FS | 1.0 | 88.5 | 111.1 |
| Example 8-9 | Nonaqueous electrolyte solution 8-9 | (9a) | 1.0 | FS | 1.0 | 70.5 | 101.9 |
| Example 8-10 | Nonaqueous electrolyte solution 8-10 | (10a) | 1.0 | FS | 1.0 | 71.2 | 102.1 |
| Example 8-11 | Nonaqueous electrolyte solution 8-11 | (11a) | 1.0 | FS | 1.0 | 76.7 | 109.8 |
| Example 8-12 | Nonaqueous electrolyte solution 8-12 | (12a) | 1.0 | FS | 1.0 | 75.8 | 112.5 |
| Comparative Example 8-1 | Comparative nonaqueous electrolyte solution 8-1 | - | - | FS | 1.0 | 100 | 100 |
| Comparative Example 8-2 | Comparative nonaqueous electrolyte solution 8-2 | DBDECP | 1.0 | FS | 1.0 | 123.5 | 95.9 |
| Comparative Example 8-3 | Comparative nonaqueous electrolyte solution 8-3 | LEDOPP | 1.0 | FS | 1.0 | 102.9 | 101.5 |

**[Table 9]**

| Table 9 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 9-1 | Nonaqueous electrolyte solution 1-1 | (1a) | 0.05 | - | - | 93.8 | 100.3 |
| Example 9-2 | Nonaqueous electrolyte solution 1-2 | (1a) | 0.1 | - | - | 88.1 | 100.9 |
| Example 9-3 | Nonaqueous electrolyte solution 1-3 | (1a) | 0.5 | - | - | 78.5 | 104.2 |
| Example 9-4 | Nonaqueous electrolyte solution 1-4 | (1a) | 1.0 | - | - | 74.2 | 105.9 |
| Example 9-5 | Nonaqueous electrolyte solution 1-5 | (1a) | 2.0 | - | - | 80.9 | 106.1 |
| Example 9-6 | Nonaqueous electrolyte solution 1-6 | (1a) | 5.0 | - | - | 97.8 | 103.2 |
| Example 9-7 | Nonaqueous electrolyte solution 1-7 | (2a) | 0.5 | - | - | 77.0 | 104.9 |
| Example 9-8 | Nonaqueous electrolyte solution 1-8 | (2a) | 1.0 | - | - | 77.8 | 107.5 |
| Example 9-9 | Nonaqueous electrolyte solution 1-9 | (3a) | 0.5 | - | - | 73.5 | 103.2 |
| Example 9-10 | Nonaqueous electrolyte solution 1-10 | (3a) | 1.0 | - | - | 70.1 | 102.4 |
| Example 9-11 | Nonaqueous electrolyte solution 1-11 | (4a) | 0.5 | - | - | 78.9 | 105.7 |
| Example 9-12 | Nonaqueous electrolyte solution 1-12 | (4a) | 1.0 | - | - | 78.1 | 107.2 |
| Comparative Example 9-1 | Comparative nonaqueous electrolyte solution 1-1 | - | - | - | - | 100 | 100 |
| Comparative Example 9-2 | Comparative nonaqueous electrolyte solution 1-2 | DBDECP | 0.5 | - | - | 120.5 | 97.9 |
| Comparative Example 9-3 | Comparative nonaqueous electrolyte solution 1-3 | DBDECP | 1.0 | - | - | 145.1 | 95.1 |
| Comparative Example 9-4 | Comparative nonaqueous electrolyte solution 1-4 | LEDOPP | 0.5 | - | - | 99.8 | 99.7 |
| Comparative Example 9-5 | Comparative nonaqueous electrolyte solution 1-5 | LEDOPP | 1.0 | - | - | 100.1 | 100.2 |

**[Table 10]**

| Table 10 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 10-1 | Nonaqueous electrolyte solution 2-1 | (1a) | 1.0 | VC | 1.0 | 78.9 | 107.1 |
| Example 10-2 | Nonaqueous electrolyte solution 2-2 | (2a) | 1.0 | VC | 1.0 | 75.5 | 109.3 |
| Example 10-3 | Nonaqueous electrolyte solution 2-3 | (3a) | 1.0 | VC | 1.0 | 67.1 | 103.2 |
| Example 10-4 | Nonaqueous electrolyte solution 2-4 | (4a) | 1.0 | VC | 1.0 | 73.1 | 106.7 |
| Comparative Example 10-1 | Comparative nonaqueous electrolyte solution 2-1 | - | - | VC | 1.0 | 100 | 100 |
| Comparative Example 10-2 | Comparative nonaqueous electrolyte solution 2-2 | DBDECP | 1.0 | VC | 1.0 | 135.1 | 94.2 |
| Comparative Example 10-3 | Comparative nonaqueous electrolyte solution 2-3 | LEDOPP | 1.0 | VC | 1.0 | 103.4 | 99.1 |

**[Table 11]**

| Table 11 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 11-1 | Nonaqueous electrolyte solution 3-1 | (1a) | 1.0 | BOB | 1.0 | 75.1 | 106.2 |
| Example 11-2 | Nonaqueous electrolyte solution 3-2 | (2a) | 1.0 | BOB | 1.0 | 72.3 | 108.8 |
| Example 11-3 | Nonaqueous electrolyte solution 3-3 | (3a) | 1.0 | BOB | 1.0 | 69.9 | 101.5 |
| Example 11-4 | Nonaqueous electrolyte solution 3-4 | (4a) | 1.0 | BOB | 1.0 | 74.5 | 107.5 |
| Comparative Example 11-1 | Comparative nonaqueous electrolyte solution 3-1 | - | - | BOB | 1.0 | 100 | 100 |
| Comparative Example 11-2 | Comparative nonaqueous electrolyte solution 3-2 | DBDECP | 1.0 | BOB | 1.0 | 131.2 | 96.8 |
| Comparative Example 11-3 | Comparative nonaqueous electrolyte solution 3-3 | LEDOPP | 1.0 | BOB | 1.0 | 99.8 | 100.2 |

**[Table 12]**

| Table 12 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 12-1 | Nonaqueous electrolyte solution 4-1 | (1a) | 1.0 | DFBOP | 1.0 | 68.7 | 105.3 |
| Example 12-2 | Nonaqueous electrolyte solution 4-2 | (2a) | 1.0 | DFBOP | 1.0 | 67.9 | 105.9 |
| Example 12-3 | Nonaqueous electrolyte solution 4-3 | (3a) | 1.0 | DFBOP | 1.0 | 65.1 | 104.2 |
| Example 12-4 | Nonaqueous electrolyte solution 4-4 | (4a) | 1.0 | DFBOP | 1.0 | 69.8 | 106.8 |
| Comparative Example 12-1 | Comparative nonaqueous electrolyte solution 4-1 | - | - | DFBOP | 1.0 | 100 | 100 |
| Comparative Example 12-2 | Comparative nonaqueous electrolyte solution 4-2 | DBDECP | 1.0 | DFBOP | 1.0 | 130.1 | 96.8 |
| Comparative Example 12-3 | Comparative nonaqueous electrolyte solution 4-3 | LEDOPP | 1.0 | DFBOP | 1.0 | 105.7 | 101.9 |

**[Table 13]**

| Table 13 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 13-1 | Nonaqueous electrolyte solution 5-1 | (1a) | 1.0 | TFOP | 1.0 | 69.2 | 104.6 |
| Example 13-2 | Nonaqueous electrolyte solution 5-2 | (2a) | 1.0 | TFOP | 1.0 | 67.7 | 106.8 |
| Example 13-3 | Nonaqueous electrolyte solution 5-3 | (3a) | 1.0 | TFOP | 1.0 | 63.8 | 101.5 |
| Example 13-4 | Nonaqueous electrolyte solution 5-4 | (4a) | 1.0 | TFOP | 1.0 | 67.6 | 108.8 |
| Comparative Example 13-1 | Comparative nonaqueous electrolyte solution 5-1 | - | - | TFOP | 1.0 | 100 | 100 |
| Comparative Example 13-2 | Comparative nonaqueous electrolyte solution 5-2 | DBDECP | 1.0 | TFOP | 1.0 | 131.2 | 93.1 |
| Comparative Example 13-3 | Comparative nonaqueous electrolyte solution 5-3 | LEDOPP | 1.0 | TFOP | 1.0 | 105.9 | 97.5 |

**[Table 14]**

| Table 14 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 14-1 | Nonaqueous electrolyte solution 6-1 | (1a) | 1.0 | FSI | 1.0 | 75.1 | 103.5 |
| Example 14-2 | Nonaqueous electrolyte solution 6-2 | (2a) | 1.0 | FSI | 1.0 | 73.3 | 107.5 |
| Example 14-3 | Nonaqueous electrolyte solution 6-3 | (3a) | 1.0 | FSI | 1.0 | 68.2 | 103.4 |
| Example 14-4 | Nonaqueous electrolyte solution 6-4 | (4a) | 1.0 | FSI | 1.0 | 74.4 | 107.2 |
| Comparative Example 14-1 | Comparative nonaqueous electrolyte solution 6-1 | - | - | FSI | 1.0 | 100 | 100 |
| Comparative Example 14-2 | Comparative nonaqueous electrolyte solution 6-2 | DBDECP | 1.0 | FSI | 1.0 | 133.3 | 95.6 |
| Comparative Example 14-3 | Comparative nonaqueous electrolyte solution 6-3 | LEDOPP | 1.0 | FSI | 1.0 | 100.0 | 100.1 |

**[Table 15]**

| Table 15 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 15-1 | Nonaqueous electrolyte solution 7-1 | (1a) | 1.0 | DFP | 1.0 | 68.9 | 104.9 |
| Example 15-2 | Nonaqueous electrolyte solution 7-2 | (2a) | 1.0 | DFP | 1.0 | 65.5 | 106.9 |
| Example 15-3 | Nonaqueous electrolyte solution 7-3 | (3a) | 1.0 | DFP | 1.0 | 65.0 | 102.2 |
| Example 15-4 | Nonaqueous electrolyte solution 7-4 | (4a) | 1.0 | DFP | 1.0 | 66.7 | 107.4 |
| Comparative Example 15-1 | Comparative nonaqueous electrolyte solution 7-1 | - | - | DFP | 1.0 | 100 | 100 |
| Comparative Example 15-2 | Comparative nonaqueous electrolyte solution 7-2 | DBDECP | 1.0 | DFP | 1.0 | 125.9 | 94.1 |
| Comparative Example 15-3 | Comparative nonaqueous electrolyte solution 7-3 | LEDOPP | 1.0 | DFP | 1.0 | 100.1 | 100.7 |

**[Table 16]**

| Table 16 | Nonaqueous electrolyte solution | Component (I) or comparative compound | | Another additive | | Initial resistance (relative value) | Discharge capacity maintenance rate (relative value) |
|---|---|---|---|---|---|---|---|
| | | Type | Addition amount [% by mass] | Type | Addition amount [% by mass] | | |
| Example 16-1 | Nonaqueous electrolyte solution 8-1 | (1a) | 1.0 | FS | 1.0 | 77.9 | 103.3 |
| Example 16-2 | Nonaqueous electrolyte solution 8-2 | (2a) | 1.0 | FS | 1.0 | 75.6 | 105.2 |
| Example 16-3 | Nonaqueous electrolyte solution 8-3 | (3a) | 1.0 | FS | 1.0 | 71.8 | 102.8 |
| Example 16-4 | Nonaqueous electrolyte solution 8-4 | (4a) | 1.0 | FS | 1.0 | 75.4 | 108.1 |
| Example 16-5 | Nonaqueous electrolyte solution 8-5 | (5a) | 1.0 | FS | 1.0 | 79.5 | 109.7 |
| Example 16-6 | Nonaqueous electrolyte solution 8-6 | (6a) | 1.0 | FS | 1.0 | 83.2 | 101.1 |
| Examples 16-7 | Nonaqueous electrolyte solution 8-7 | (7a) | 1.0 | FS | 1.0 | 77.7 | 106.9 |
| Examples 16-8 | Nonaqueous electrolyte solution 8-8 | (8a) | 1.0 | FS | 1.0 | 87.1 | 109.8 |
| Examples 16-9 | Nonaqueous electrolyte solution 8-9 | (9a) | 1.0 | FS | 1.0 | 69.1 | 100.5 |
| Example 16-10 | Nonaqueous electrolyte solution 8-10 | (10a) | 1.0 | FS | 1.0 | 68.3 | 101.7 |
| Example 16-11 | Nonaqueous electrolyte solution 8-11 | (11a) | 1.0 | FS | 1.0 | 77.9 | 108.7 |
| Example 16-12 | Nonaqueous electrolyte solution 8-12 | (12a) | 1.0 | FS | 1.0 | 76.1 | 110.3 |
| Comparative Example 16-1 | Comparative nonaqueous electrolyte solution 8-1 | - | - | FS | 1.0 | 100 | 100 |
| Comparative Example 16-2 | Comparative nonaqueous electrolyte solution 8-2 | DBDECP | 1.0 | FS | 1.0 | 141.1 | 96.1 |
| Comparative Example 16-3 | Comparative nonaqueous electrolyte solution 8-3 | LEDOPP | 1.0 | FS | 1.0 | 108.9 | 98.3 |

As is clear from Tables 1 to 16, it has been found that a nonaqueous electrolyte solution battery using a nonaqueous electrolyte solution including the component (I) of the present disclosure had low initial resistance. Further, it has been found that the nonaqueous electrolyte solution using a compound containing at least one carbon-carbon unsaturated bond as the component (I) of the present disclosure achieved an excellent high-temperature cycle characteristic.

### INDUSTRIAL APPLICABILITY

The present disclosure can provide a nonaqueous electrolyte solution and a nonaqueous electrolyte solution battery having a low initial resistance value, and a method for producing a nonaqueous electrolyte solution battery having a low initial resistance value. In addition, the present disclosure can provide a compound and an additive for a nonaqueous electrolyte solution, both of which can be suitably used in the above nonaqueous electrolyte solution.

Although the present disclosure has been described in detail and with reference to specific examples, it will be apparent to those skilled in the art that various changes and modifications can be made without departing from the spirit and scope of the present disclosure.

The present application is based on a Japanese Patent Application (No. 2022-016731) filed on February 4, 2022, the contents of which are incorporated herein by reference.

## Claims

1. A nonaqueous electrolyte solution comprising:
(I) at least one selected from the group consisting of a compound represented by the following Formula (1) and a compound represented by the following Formula (2): 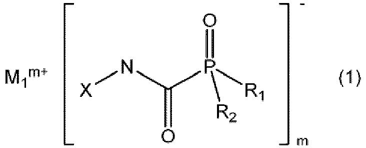
wherein R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R₁ and R₂ optionally bond to each other to form a cyclic structure,
X represents a monovalent substituent, and
M₁^{m+} represents a proton, a metal cation, or an onium cation, and m represents a valence of the cation; 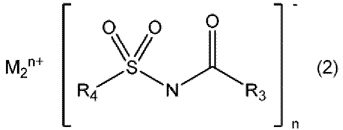
wherein R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom,
R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
M₂ⁿ⁺ represents a proton, a metal cation, or an onium cation, and n represents a valence of the cation.

2. The nonaqueous electrolyte solution according to claim 1, wherein X is -C(=O)-Rₐ, -S(=O)₂-R_{b}, or -P(=O)-R_{c}R_{d}, and Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms, and
at least one hydrogen atom in the organic group is optionally substituted with a halogen atom, and R_{c} and R_{d} optionally bond to each other to form a cyclic structure.

3. The nonaqueous electrolyte solution according to claim 1, wherein X represents -S(=O)₂F or -P(=O)F₂.

4. The nonaqueous electrolyte solution according to claim 1, wherein at least one selected from the group consisting of M₁^{m+} in the Formula (1) and M₂ⁿ⁺ in the Formula (2) represents a lithium ion, a sodium ion, or a proton.

5. The nonaqueous electrolyte solution according to claim 1, wherein at least one selected from the group consisting of the compound represented by the Formula (1) and the compound represented by the Formula (2) contains at least one carbon-carbon unsaturated bond.

6. The nonaqueous electrolyte solution according to any one of claims 1 to 5,
wherein the nonaqueous electrolyte solution contains a (II) solute, and the (II) solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCla, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

7. The nonaqueous electrolyte solution according to any one of claims 1 to 5,
wherein the nonaqueous electrolyte solution contains a (III) nonaqueous organic solvent, and the (III) nonaqueous organic solvent contains at least one selected from the group consisting of a cyclic carbonate and a chain carbonate.

8. The nonaqueous electrolyte solution according to claim 7, wherein the cyclic carbonate is at least one selected from the group consisting of ethylene carbonate, propylene carbonate, and fluoroethylene carbonate, and the chain carbonate is at least one selected from the group consisting of ethyl methyl carbonate, dimethyl carbonate, diethyl carbonate, and methyl propyl carbonate.

9. The nonaqueous electrolyte solution according to any one of claims 1 to 5, wherein a content of the (I) is 0.01% by mass to 10.0% by mass with respect to a total amount of the nonaqueous electrolyte solution.

10. The nonaqueous electrolyte solution according to any one of claims 1 to 5, further comprising:
at least one selected from vinylene carbonate, bis(oxalato)borate, difluorooxalatoborate, difluorobis(oxalato)phosphate, tetrafluorooxalatophosphate, bis(fluorosulfonyl)imide salt, bis(difluorophosphoryl)imide salt, (difluorophosphoryl)(fluorosulfonyl)imide salt, difluorophosphate, fluorosulfonate, methanesulfonyl fluoride, 1,3,2-dioxathiolane-2,2-dioxide, 1,3-propenesultone, 1,3-propanesultone, 1,6-diisocyanatohexane, ethynylethylene carbonate, 4-propyl-1,3,2-dioxathiolane-2,2-dioxide, methylene methanedisulfonate, 1,2-ethanedisulfonic anhydride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.

11. A nonaqueous electrolyte solution battery comprising:
at least a positive electrode, a negative electrode, and the nonaqueous electrolyte solution according to any one of claims 1 to 5.

12. A method for producing a nonaqueous electrolyte solution battery, the method comprising:
preparing the nonaqueous electrolyte solution according to any one of claims 1 to 5; and
filling an empty cell including a positive electrode and a negative electrode with the nonaqueous electrolyte solution.

13. A compound represented by the following Formula (1A): 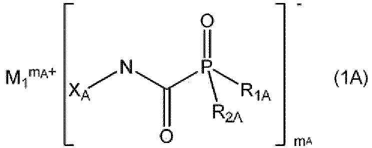
wherein R_{1A} and R_{2A} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, R_{1A} and R_{2A} optionally bond to each other to form a cyclic structure,
X_{A} represents a monovalent substituent, and
M₁^{mA+} represents a metal cation or an onium cation, and m_{A} represents a valence of the cation.

14. The compound according to claim 13, wherein M₁^{mA+} represents a lithium ion or a sodium ion.

15. The compound according to claim 13, wherein the compound represented by the Formula (1A) contains at least one carbon-carbon unsaturated bond.

16. A compound represented by the following Formula (1B): 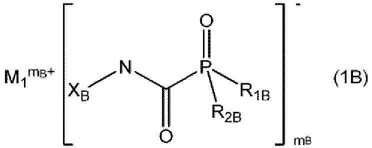
wherein R_{1B} and R_{2B} each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R_{1B} and R_{2B} optionally bond to each other to form a cyclic structure,
X_{B} represents a monovalent substituent,
M₁^{mB+} represents a proton, a metal cation, or an onium cation, and m_{B} represents a valence of the cation, and
the compound represented by Formula (1B) contains at least one carbon-carbon unsaturated bond.

17. The compound according to any one of claims 13 to 16,
wherein at least one selected from the group consisting of X_{A} in the Formula (1A) and X_{B} in the Formula (1B) is - C(=O)-Rₐ, -S(=O)₂-R_{b}, or -P(=O)-R_{c}R_{d}, and Rₐ to R_{d} each independently represent a fluorine atom or an organic group selected from the group consisting of an alkyl group having 1 to 8 carbon atoms, an alkoxy group having 1 to 8 carbon atoms, an alkenyl group having 2 to 6 carbon atoms, an alkenyloxy group having 2 to 6 carbon atoms, an alkynyl group having 3 to 6 carbon atoms, an alkynyloxy group having 3 to 6 carbon atoms, an aryl group having 6 to 12 carbon atoms, and an aryloxy group having 6 to 12 carbon atoms, and
at least one hydrogen atom in the organic group is optionally substituted with a halogen atom, and R_{c} and R_{d} optionally bond to each other to form a cyclic structure.

18. The compound according to any one of claims 13 to 16, wherein at least one selected from the group consisting of X_{A} in the Formula (1A) and X_{B} in Formula (1B) represents -S(=O)₂F or -P(=O)F₂.

19. A compound represented by the following Formula (2C): 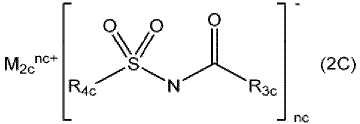
wherein R_{3C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom,
R_{4C} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom, and
M_{2C}^{nC+} represents a proton, a metal cation, or an onium cation, and nc represents a valence of the cation.

20. A compound represented by the following Formula (2D): 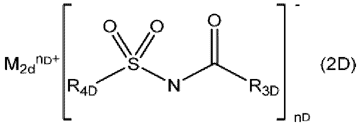
wherein R_{3D} represents an organic group selected from the group consisting of an alkenyloxy group having 2 to 10 carbon atoms, an alkynyloxy group having 2 to 10 carbon atoms, and a cycloalkenyloxy group having 3 to 10 carbon atoms, and at least one hydrogen atom in the organic group is optionally substituted with a halogen atom,
R_{4D} represents a fluorine atom, and
M_{2d}^{nD+} represents a proton, a metal cation, or an onium cation, and n_{D} represents a valence of the cation.

21. The compound according to claim 19, wherein M_{2C}^{nC+} in the Formula (2C) represents a lithium ion, a sodium ion, or a proton.

22. The compound according to claim 20, wherein M_{2d}^{nD+} in the Formula (2D) represents a lithium ion or a sodium ion.

23. An additive for a nonaqueous electrolyte solution, comprising:
at least one selected from the group consisting of a compound represented by the following Formula (1) and a compound represented by the following Formula (2): 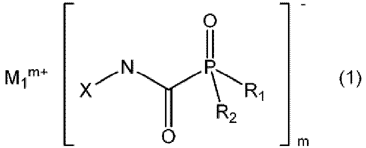
wherein R₁ and R₂ each independently represent an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group is optionally substituted with a halogen atom, and R₁ and R₂ optionally bond to each other to form a cyclic structure,
X represents a monovalent substituent, and
M₁^{m+} represents a proton, a metal cation, or an onium cation, and m represents a valence of the cation; 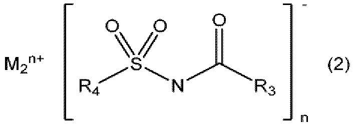
wherein R₃ represents an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom,
R₄ represents a halogen atom, or an organic group selected from the group consisting of an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, and at least one hydrogen atom in the organic group is optionally substituted with a halogen atom, and
M₂ⁿ⁺ represents a proton, a metal cation, or an onium cation, and n represents a valence of the cation.
